# EUROPEAN PATENT APPLICATION

(11) **EP 1 333 091 A1**
(43) Date of publication of application: **06.08.2003**
(21) Application number: 01976732.6
(22) Date of filing: 18.10.2001
(51) Int. Cl.: C12N 15/12, C07K 14/47, C07K 16/18, C12P 21/02, C12N 1/15, C12N 1/19

(54) **NOVEL DISEASE-ASSOCIATED GENE AND USE THEREOF**

(30) Priority: 19.10.2000 JP 2000319912; 16.11.2000 JP 2000350183
(71) Applicant: Takeda Chemical Industries, Ltd., Osaka-shi, Osaka 541-0045 (JP)
(72) Inventor: KOYAMA, Nobuyuki, Takeda Kasuga Haitsu 202, Tsukuba-shi, Ibaraki 305-0821 (JP); TANIDA, Seiichi, Nagaokakyo-shi, Kyoto 617-0857 (JP); WATANABE, Toshifumi, Kawachinagano-shi, Osaka 586-0092 (JP)
(74) Representative: Rickard, Timothy Mark Adrian
(86) International application number: JP0109140
(87) International publication number: WO02033082

(57) **Abstract**

It is an object of the present invention to provide a novel disease-associated gene and use of the protein thereof, etc. Specifically, the invention provides: (1) a screening method and a screening kit using the protein and a salt thereof; (2) a compound or a salt thereof controlling an activity of the protein, obtained by the screening method and the screening kit; (3) a neutralizing antibody regulating the activity of the protein; and (4) a medicine such as a preventive and a remedy for a heart disease.

## Description

### TECHNICAL FIELD

The present invention relates to a disease- (e.g. heart disease-) associated gene, a protein thereof and its salt, a polynucleotide encoding it, a method of manufacturing the protein or its salt and its partial peptide, a recombinant vector and a transformant comprising the polynucleotide, a screening method and a screening kit using the protein and the salt thereof, a compound or a salt thereof obtained by using the screening method and screening kit, and the like.

### TECHNICAL BACKGROUND

Heart failure is considered as insufficient myocardial contraction. Possible onset mechanisms of heart failure is as follows: disturbance of a heart muscle, mechanical and functional abnormality of a heart pump, an excessive pressure loaded by hypertension and pulmonary hypertension, a voluminal overload due to anemia or acute nephritis to cause a condition of an impossible cardiac output of a blood volume sufficient to a living body demand. For this condition, compensation mechanisms by a sympathetic nerve system, nerves - body fluid - endocrine system, and the like start to work for maintaining the physiological homoeostasis. In the compensation mechanisms of heart failure, 1) an increase in the aforementioned loads causes an increase in cardiac contraction force, resulting in cardiac dilatation along with an extension of a sarcomere, 2) a myocardial contraction unit extends to cause myocardial hypertrophy, and 3) a neurohumoral factor is activated to compensate for the condition of the insufficient cardiac output of blood systemically required to develop local myocardial fibrosis. Fundamentally, these are mechanisms for adaptation to the given loads; however, there is a case of heart failure enhanced by an insufficient action of the mechanisms and another case of heart failure become worse by the excessive action. The action of the compensation mechanisms results in myocardial cell hypertrophy to cause hypercardia. However, when the aforementioned disturbances or loads continue chronically, the compensation mechanisms will fail. In this case, the enlarged myocardial cell cannot receive a sufficient volume of blood to cause ischemia, resulting in myocardial disturbances such as insufficient myocardial contraction and finally bringing about heart failure syndrome accompanied by such as lowering of the cardiac output, cardiovascular disorder, venous stasis, retention of fluid. For treatment of the syndrome, it is necessary to improve the myocardial cell disturbance, reinforce the cardiac protecting action, recover from the cardiac hypofunction caused by insufficient myocardial contraction, and prevent the underlying failure of the biological compensation or improve an excessive compensation.

Drugs currently used for treatment of the heart failure syndromes are clinically, cardiotonic drugs such as 1) a cardiotonic glycoside such as digoxin, 2) a sympathetic agent such as dobutamine, 3) a phosphodiesterase inhibiter such as amrinone, and vasodilatator drugs such as hydralazine, calcium antagonist, angiotensin I-converting enzyme inhibitor, and angiotensin II receptor antagonist. For treatment of dilated cardiomyopathy, β-blocker is used.

However, there is no remedy for quickly starting the compensation mechanisms and suppressing the excessive expression of compensation mechanisms or suppressing compensation failure including apoptosis. We intended to develop the remedy for the quick start of the compensation mechanisms and suppression of the excessive compensation or the compensation failure.

### DISCLOSURE OF THE INVENTION

The inventors intensively studied to solve the aforementioned problem, targeting a gene, of which expression rises in a heart at the onset of heart failure. As the result of the study, they found that an mRNA (SEQ ID No: 2) increases its expression at the onset of heart failure in a model rat of cardiac infarction prepared by a surgery to ligate the coronary artery. A detailed examination of an expression profile of the mRNA showed that this mRNA was increased slightly 1 week after the surgery, reduced 8 weeks after the surgery, and also increased distinctly 20 and 30 weeks after the surgery. Homology test of this base sequence to that of a known gene showed that this is the gene indicating a high homology to a mouse G protein signal regulator. A further study based on these findings made us achieve the invention. A compound obtained by the screening method and the screening kit using a protein, which is a product of the gene of the invention, and a salt thereof or a partial peptide thereof is useful as a preventive or therapeutic method and a diagnostic reagent and a pharmaceutical composition for heart failure or diseases caused by the gene. In addition, the preparation of the DNA-transfected animal and knockout animal of the invention is also useful for causal analysis of heart failure and the protein-associated diseases and test of therapeutic methods. Hence, the present invention also provides the method of screening for a compound regulating an activity of the protein, a compound obtained by the screening method and a salt thereof, a pharmaceutical composition comprising the salt, and the DNA-transfected animal and knockout animal of the invention.

The invention provides:
(1) A protein having the same or substantially the same amino acid sequence as the amino acid sequence shown by SEQ ID NO: 1, or a salt thereof;
(2) A protein comprising the amino acid sequence shown by SEQ ID NO: 1, or a salt thereof;
(3) A partial peptide of the protein according to (1), or a salt thereof;
(4) A polynucleotide comprising a polynucleotide encoding the protein according to (1) or its salt, or the partial peptide according to (3) or its salt;
(5) The polynucleotide according to (4), which is a DNA;
(6) The DNA according to (5), which comprises the base sequence represented by SEQ ID NO: 2;
(7) A recombinant vector comprising the polynucleotide according to (4);
(8) A transformant transformed by the recombinant vector according to (7);
(9) A method of manufacturing the protein according to (1) or its salt or the partial peptide according to (3) or its salt, which comprises culturing the transformant according to (8) to produce and accumulate the protein according to (1) or its salt or the partial peptide according to (3) or its salt; and collecting it;
(10) A pharmaceutical composition comprising the protein according to (1) or its salt, or the partial peptide according to (3) or its salt;
(11) An antibody to the protein according to (1) or its salt, or the partial peptide according to (3) or its salt;
(12) A diagnostic agent comprising the antibody according to (11);
(13) A method of screening for a compound or its salt regulating an activity of the protein according to (1) or its salt, or the partial peptide according to (3) or its salt, which comprises using the protein according to (1) or its salt, or the partial peptide according to (3) or its salt;
(14) The screening method according to (13), wherein the protein according to (1) or its salt, or the partial peptide according to (3) or its salt is expressed in a cytoplasm of a transformant transformed by a DNA comprising a DNA encoding the protein according to (1) or its salt or the partial peptide according to (3) or its salt;
(15) A kit for screening for a compound or its salt regulating the activity of the protein according to (1) or its salt, or the partial peptide according to (3) or its salt, which comprises the protein according to (1) or its salt, or the partial peptide according to (3) or its salt;
(16) A compound or its salt regulating the activity of the protein according to (1) or its salt, or the partial peptide according to (3) or its salt, which is obtained by the screening method according to (13) or the screening kit according to (15);
(17) A pharmaceutical composition comprising the compound according to (16) or its salt;
(18) The pharmaceutical composition according to (17), which is a preventive and/or therapeutic agent for a heart disease;
(19) An antisense nucleotide having a base sequence complementary or substantially complementary to a DNA encoding the protein according to (1) or its salt or the partial peptide according to (3) or its salt;
(20) A pharmaceutical composition comprising the antisense nucleotide according to (19);
(21) A pharmaceutical composition comprising the polynucleotide according to (4);
(22) A diagnostic agent comprising the polynucleotide according to (4);
(23) A preventive and/or therapeutic method for a heart disease in a mammal, which comprises administering an effective amount of the compound according to (16) or its salt to the mammal;
(24) A use of the compound according to (16) or its salt for manufacturing a preventive and/or therapeutic agent for a heart disease.

Furthermore, the invention provides:
(25) A non-human, DNA-transfected animal having the DNA encoding the protein according to (1) or its mutant DNA;
(26) The animal according to (25), wherein the non-human animal is a rodent;
(27) The animal according to (26), wherein the rodent is a mouse or a rat;
(28) An embryonic stem cell of a non-human mammal, in which the DNA of the invention is inactivated;
(29) The embryonic stem cell according to (28), in which the DNA is inactivated by introducing a reporter gene (e.g., β-galactosidase gene derived from Escherichia coli);
(30) The embryonic stem cell according to (28), which is resistant to neomycin;
(31) The embryonic stem cell according to (28), wherein the non-human mammal is a rodent;
(32) The embryonic stem cell according to (31), wherein the rodent is a mouse;
(33) A non-human mammal deficient in expressing the DNA of the invention, in which the DNA of the invention is inactivated;
(34) The non-human mammal according to (32), in which the DNA is inactivated by introducing a reporter gene (e.g., β-galactosidase gene derived from Escherichia coli) therein and the reporter gene can be expressed under the control of a promoter for the DNA of the invention;
(35) The non-human mammal according to (32), which is a rodent;
(36) The non-human mammal according to (35), wherein the rodent is a mouse; and
(37) A method of screening for a compound or its salt enhancing or inhibiting the promoter activity for the DNA of the invention, which comprises administering a test compound to the animal according to (36) and detecting the expression of the reporter gene.

### BRIEF DESCRIPTION OF THE DRAWINGS

Fig. 1 shows a tissue distribution of the gene of the invention, in a healthy rat by northern blot analysis.
Fig. 2 shows changes in the expression of cDNA having the base sequence represented by SEQ ID No: 2 with time in a rat model of cardiac infarction.

### BEST MODE FOR CARRYING OUT THE INVENTION

The protein (hereinafter may be referred to the protein of the invention or the protein used in the invention) comprising the same or substantially the same amino acid sequence as the amino acid sequence represented by SEQ ID NO: 1 may be any protein derived from any cells of human or any other warm-blooded animals (e.g., guinea pig, rat, mouse, chicken, rabbit, swine, sheep, bovine, monkey, etc.) (e.g., hepatocytes, splenocytes, nerve cells, glial cells, cells of pancreas, bone marrow cells, mesangial cells, Langerhans' cells, epidermic cells, epithelial cells, beaker cell, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.); or any tissues where such cells are present, such as brain or any of brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; the proteins may also be synthetic proteins.

The amino acid sequence which is substantially the same as the amino acid sequence represented by SEQ ID NO: 1 includes amino acid sequences having at least about 97% homology, preferably at least about 98% homology, and more preferably at least about 99% homology to the amino acid sequence represented by SEQ ID NO:1.

As a protein comprising substantially the same amino acid sequence as the amino acid sequence represented by SEQ ID NO: 1, for example, a protein comprising substantially the same amino acid sequence as the amino acid sequence represented by SEQ ID NO: 1 and having substantially the same activity in quality as that of a protein having the amino acid sequence represented by SEQ ID NO: 1 is preferred.

Such an activity having substantially the same quality is exemplified by the activity of enhancing the cardiac hypofunction. "Substantially the same quality" means that the activity is identical in quality (e.g., physiologically or pharmacologically). Hence, it is preferable that the activity of enhancing the cardiac hypofunction is the same level (e.g., about 0.01 to 100 folds, preferably about 0.1 to 10 folds, more preferably about 0.5 to 2 folds), however, quantitative factors such as a level of this activity and a molecular weight of a protein may be different.

The activity of enhancing the cardiac hypofunction can be measured by an echocardiographic device (Cell, Vol. 97: 189-198, 1999) or cardiac function measurement using a cardiac catheter (Circulation Research 69: 370-377, 1991). Moreover, the said activity can be determined by, for example, measurement of activation of renin-angiotensin system (RAS) such as angiotensin I-converting enzyme (ACE) using a commercial kit (e.g., made by Peninsula Corp., Phoenix Corp., etc.) or measurement of an increase in blood catecholamine (full automatic catecholamine analyzer, Toso) as an index.

The proteins of the invention also include so-called muteins such as proteins comprising (i) an amino acid sequence represented by SEQ ID NO: 1, of which one or more (preferably 1 to 5) amino acids are deleted; (ii) an amino acid sequence represented by SEQ ID NO: 1, to which one or more (preferably approximately 1 to 30, more preferably approximately 1 to 10, and even more preferably 1 to 5) amino acids are added; (iii) an amino acid sequence represented by SEQ ID NO: 1, into which one or more (preferably 1 to 5) amino acids are inserted, (iv) an amino acid sequence represented by SEQ ID NO: 1, in which one or more (preferably 1 to 5) amino acids are substituted by other amino acids; and (v) a combination of the above amino acid sequences.

Throughout the present specification, the proteins are represented in accordance with the conventional way of describing peptides, that is, with the N-terminus (amino terminus) on the left side and the C-terminus (carboxyl terminus) on the right side. In the proteins of the invention including the protein having the amino acid sequence shown by SEQ ID NO: 1, the C-terminus may be in the form of a carboxyl group (-COOH), a carboxylate (-COO⁻), an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc., and an α-naphthyl-C₁₋₂ alkyl group, e.g., α-naphthylmethyl, etc.; pivaloyloxymethyl, and the like.

When the protein of the invention contains a carboxyl group (or a carboxylate) at a position other than the C-terminus, it may be amidated or esterified and such an amide or ester is also included within the protein of the invention. The ester group may be the same group as that described with respect to the above C-terminal group.

Furthermore, examples of the protein of the invention include variants of the above proteins, wherein the amino group at the N-terminus (e.g., methionine residue) of the protein is protected with a protecting group (e.g., a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins having sugar chains.

The protein of the invention is specifically exemplified by the protein derived from a rat, which contains the amino acid sequence represented by SEQ ID NO: 1.

The partial peptides of the protein of the invention (hereafter occasionally referred to as the partial peptides of the invention; the term, partial peptides of the invention, may be used including amides and esters thereof) may be any partial peptides of the protein of the invention described above, preferably those having a similar activity as that of the protein of the invention described above.

For example, there are employed peptides having sequences of at least 20, preferably at least 50, more preferably at least 70, much more preferably at least about 100 and most preferably at least about 150 amino acids in the amino acid sequence which constitutes the protein of the invention.

In the partial peptide of the invention, one or more (preferably 1 to 5) amino acids in the amino acid sequence of the partial peptide may be deleted; one or more (preferably approximately 1 to 20, more preferably approximately 1 to 10, and even more preferably several 1 to 5) amino acids may be added to the amino acid sequence; one or more (preferably 1 to 5) amino acids may be inserted into the amino acid sequence; or one or more (preferably 1 to 5) amino acids in the amino acid sequence may be substituted by other amino acids.

A C-terminal of the partial peptides of the invention may be any one of carboxyl (-COOH,) carboxylate (-COO⁻,) amide (-CONH₂,) or ester (-COOR) (R means the same definition as described above).

The partial peptides of the invention include, as in the aforementioned protein of the invention, those in which the amino group of N-terminal amino acid residue (e.g., methionine residue) has been protected by a protecting group, those in which glutamine residue, produced by cleavage of the N-terminal region in vivo, has been pyroglutaminated, those in which a substituent in a side chain of the amino acid in the molecule has been protected by a suitable protecting group, or a conjugated peptide such as so-called glycopeptide which has a sugar chain.

The partial peptide of the invention is usable as an antigen for producing antibodies thereto.

The salt of the protein or its partial peptide of the invention may be a salt with physiologically acceptable acids (e.g., inorganic acids, organic acids) or bases (e.g., alkaline metal salts), and physiologically acceptable acid addition salts are particularly preferred. Examples of such salts are salts with inorganic acids (e.g., hydrochloric acid, phosphoric acid, hydrobromic acid or sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or its partial peptide or its salt of the invention may be manufactured by publicly known methods used to purify proteins from human or any other warm-blooded animal tissues or cells described above, or may be manufactured by culturing transformants containing DNA encoding the protein. The protein or its salt may also be manufactured by the peptide synthesis methods later described.

When the protein or its partial peptide is manufactured from human or any other warm-blooded animal tissues or cells, these tissues or cells are homogenized, then the protein or its partial peptide is extracted with an acid or the like, and is isolated and purified from the extract by a combination of chromatography techniques such as reversed phase chromatography, ion exchange chromatography, and the like.

To synthesize the protein of the invention or the partial peptide, or salts thereof, or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known. At the end of the reaction, the protein is excised from the resin and at the same time, the protecting groups are removed. Then, intramolecular disulfide bond-forming reaction is performed in a highly diluted solution to obtain the objective protein or amides thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents used to activate the protected amino acids or condense with the resin may be chosen from solvents that are known to be usable for protein condensation reactions. Acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents are usable. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As the activated form of amino groups in the starting material, the corresponding phosphoric amide is employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein or the partial peptide, for example, the α-carboxyl group of the carboxy terminal amino acid is first protected by amidation; the peptide (protein) chain is then extended to amino group for a desired length. Thereafter, the protein or the partial peptide in which only the protecting group of the N-terminal α-amino group has been eliminated from the protein or the partial peptide in which only the protecting group of the C-terminal carboxyl group has been eliminated are manufactured. The protein or the peptide is condensed in a mixture of the solvents described above. The details of the condensation reaction are the same as described above. After the protected protein or the peptide obtained by the condensation is purified, all the protecting groups are eliminated by the method described above to give the desired crude protein or peptide. This crude protein or peptide is purified by various known purification means. Lyophilization of the major fraction gives the desired amide of the protein or peptide.

To prepare the esterified protein or peptide, for example, the α-carboxyl group of the carboxy terminal amino acid is condensed with a desired alcohol to prepare the amino acid ester, which is then processed by procedure similar to the preparation of the amidated protein or peptide above to give the desired esterified protein or peptide.

The protein and its partial peptide of the invention or salts thereof can be manufactured by publicly known methods for peptide synthesis, or by cleaving the protein of the invention with an appropriate peptidase. For the methods for peptide synthesis, for example, either solid phase synthesis or liquid phase synthesis may be used. That is, a partial peptide or amino acids that can construct the partial peptide of the invention can be condensed with the remaining part of the partial peptide of the invention. When the product contains protecting groups, these protecting groups are removed to give the desired peptide. Publicly known methods for condensation and elimination of the protecting groups are described in the following 1) to 5.)
1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: Peptide Gosei-no-Kiso to Jikken (Basics and experiments of peptide synthesis), published by Maruzen Co. (1975)
4) Haruaki Yajima & Shunpei Sakakibara: Seikagaku Jikken Koza (Biochemical Experiment) 1, Tanpakushitsu no Kagaku (Chemistry of Proteins) IV, 205 (1977)
5) Haruaki Yajima ed.: Zoku Iyakuhin no Kaihatsu (A sequel to Development of Pharmaceuticals), Vol. 14, Peptide Synthesis, published by Hirokawa Shoten

After completion of the reaction, the partial peptide of the invention can be purified and isolated by a combination of conventional purification methods such as solvent extraction, distillation, column chromatography, liquid chromatography and recrystallization to give the partial peptide of the invention. When the protein or the partial peptide obtained by the above methods is in a free form, it can be converted into an appropriate salt by a publicly known method; when the protein or the partial peptide is obtained in a salt form, it can be converted into a free form or a different salt form by the publicly known method.

The polynucleotide encoding the protein of the invention may be any polynucleotide which contains the base sequence (DNA or RNA, preferably DNA) encoding the protein of the invention described above. The polynucleotide may be DNA or RNA such as mRNA encoding the protein of the invention and may be double-stranded or single-stranded. The double-stranded polynucleotide may be a double-stranded DNA, a double-stranded RNA, or a DNA:RNA hybrid. The single-stranded polynucleotide may be a sense strand (i.e. coding strand) or an antisense strand (i.e. non-coding strand).

Using the polynucleotide encoding the protein of the invention allows quantification of the mRNA of the protein of the invention by the known method described in, e.g. Zikken Igaku, Zoukan "New PCR and its application" 15(7), 1997 or a similar method.

The DNA encoding the protein of the invention may be any DNA containing the base sequence encoding the above-described protein of the invention. The DNA may be derived from a genome DNA, a genome DNA library, cDNAs derived from the aforementioned tissues and cells, a cDNA library derived from the aforementioned tissues and cells, or synthetic DNAs.

The vector to be used for the library may be any one of bacteriophage, plasmid, cosmid, phagemid, and the like. In addition, the DNA can be amplified by reverse transcriptase polymerase chain reaction (hereinafter abbreviated as RT-PCR) from total RNA or mRNA fraction prepared from the above-described cells or tissues.

The DNA encoding the protein of the invention may be, for example, any DNA comprising the base sequence represented by SEQ ID NO: 2, or any DNA hybridizable to the DNA having the base sequence represented by SEQ ID NO: 2 under high stringent conditions and encoding a protein which has a property substantially equivalent to the property of the protein of the invention, which has the amino acid sequence represented by SEQ ID No: 1.

Examples of the DNA that is hybridizable to the DNA comprising the base sequence represented by SEQ ID NO: 2 under high stringent conditions include DNAs comprising a base sequence with at least about 97% homology, preferably at least about 98% homology, and more preferably at least about 99% homology to the base sequence represented by SEQ ID NO: 2.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, the hybridization condition in a sodium concentration at about 19 mM at a temperature of about 65°C is most preferred.

More specifically, the DNA encoding the protein having the amino acid sequence represented by SEQ ID NO: 1 may be the DNA having the base sequence represented by SEQ ID NO: 2.

The term "polynucleotide comprising a part of the base sequence of the DNA encoding the protein of the invention or a part of the base sequence complementary to that of the DNA" means not only the DNA encoding the partial peptide of the invention described later, but also the RNA.

According to the invention, an antisense polynucleotide (nucleic acid) capable of inhibiting the replication or expression of the gene of the protein of the invention can be designed and synthesized on the basis of a base sequence information on the DNA encoding the protein, which is cloned or determined. Such polynucleotide (nucleic acid) can be hybridized with the RNA of the gene of the protein of the invention, and can inhibit synthesis or a function of the RNA or can regulate and control the expression of the gene of the protein of the invention through an interaction with an RNA associated with the protein of the invention. A polynucleotide complementary to a selected sequence of the RNA associated with the protein of the invention and a polynucleotide hybridizable specifically with the RNA associated with the protein of the invention are useful for regulating and controlling in vivo and in vitro expression of the gene of the protein of the invention and also useful for therapy and diagnosis of diseases. A term "corresponding" means "having homology" or "being complementary" to a given nucleotide sequence, a base sequence, or a nucleic acid sequence, including the gene. "Correspondence" between a peptide (protein) and a nucleotide sequence, base sequence, or nucleic acid sequence means usually that the nucleotide (nucleic acid) sequence or the complementary sequence thereto defines the amino acid sequence of the peptide (protein). Preferred target regions include a 5'-end hairpin loop, 5'-end 6-base-pair repeats, 5'-end untranslated region, polypeptide translation initiation codon, protein coding region, ORF translation termination codon, 3'-end untranslated region, 3'-end palindrome region, and 3'-end hairpin loop in the gene of the protein, but any region within the gene of the protein can be selected as the target.

The relationship between the target nucleic acid and the polynucleotide complementary to at least a part of the target region, specifically the relationship between the target and the polynucleotide hybridizable to the target, is designated to be "antisense". The antisense polynucleotide may be a polynucleotide containing 2-deoxy-D-ribose, a polynucleotide containing D-ribose, any other types of polynucleotides, including N-glycosides of purine or pyrimidine bases, or other polymers containing non-nucleotide backbones (e.g., proteinous nucleic acids and synthetic sequence-specific nucleic acid polymers commercially available) or other polymers containing non-standard linkages (wherein the polymers contain nucleotides having such a configuration that allows base pairing or base sticking, as is found in DNA and RNA). The antisense polynucleotide may be a double-stranded DNA, a single-stranded DNA, a single-stranded RNA or a DNA:RNA hybrid, and further includes unmodified polynucleotides (or unmodified oligonucleotides), and polynucleotides having publicly known types of modifications, for example, those having labels known in the art, those having caps, methylated polynucleotides, those having substitution of one or more naturally occurring nucleotides having their analogue, those having intramolecular modifications of nucleotides such as those having uncharged linkages (e.g., methyl phosphonates, phosphotriesters, phosphoramidates, carbamates, etc.) and those having charged linkages or sulfur-containing linkages (e.g., phosphorothioates, phosphorodithioates, etc.), those having side chain groups such as proteins (including nucleases, nuclease inhibitors, toxins, antibodies, signal peptides, poly-L-lysine, etc.) or saccharides (e.g., monosaccharides, etc.), those having intercalators (e.g., acridine, psoralen, etc.), those containing chelators (e.g., metals, radioactive metals, boron, oxidative metals, etc.) and those containing alkylating agents, those having modified linkages (e.g., α anomeric nucleic acids, etc.). As used herein, the terms "nucleoside," "nucleotide" and "nucleic acid" may include those containing not only purine and pyrimidine bases, but also other heterocyclic bases, which are modified. Such modifications may include methylated purines and pyrimidines, acylated purines and pyrimidines or other heterocyclic rings. Modified nucleotides and modified nucleotides may contain modifications on the sugar moiety, for example, wherein one or more hydroxyl groups may optionally be replaced with a halogen, aliphatic groups, etc., or may be converted into the corresponding functional groups such as ethers, amines, or the like.

The antisense polynucleotide (nucleic acid) of the invention is an RNA, a DNA or a modified nucleic acid (RNA, DNA). Examples of the modified nucleic acid are, but not limited to, a sulfurized derivative and a thiophosphate derivative of the nucleic acid, and those resistant to degradation of polynucleoside or oligonucleoside amides. The antisense nucleic acid of the invention can be preferably designed in such a way to increase the intracellular stability of the antisense nucleic acid, to increase the permeability of the antisense nucleic acid into cells, to increase the affinity of the antisense nucleic acid to the target sense strand, and to reduce the toxicity, if any, of the antisense nucleic acid.

Many such modifications are known in the art, as disclosed in J. Kawakami et al., Pharm. Tech. Japan, Vol. 8, pp. 247, 1992; Vol. 8, pp. 395, 1992; S. T. Crooke et al. ed., Antisense Research and Applications, CRC Press, 1993; etc.

The antisense nucleic acid of the invention may be modified or contain modified sugars, bases or linkages. The antisense nucleic acid may be provided or applied to gene therapy in a special form such as liposomes or microspheres, or may be provided in a form having attached moieties. Such attached moieties include polycations such as polylysine, which act to neutralize the charge of the phosphate backbone, or hydrophobic moieties such as lipids (e.g., phospholipids, cholesterols, etc.), which enhance the interaction with cell membranes or increase the uptake of the nucleic acid. Preferred examples of the lipids to be attached are cholesterols or derivatives thereof (e.g., cholesteryl chloroformate, cholic acid, etc.). These moieties may be attached to the nucleic acid at the 3'- or 5'-end thereof, or may be attached thereto through a base, sugar, or intramolecular nucleoside linkage. Other moieties may be capping groups specifically placed at the 3'- or 5'-end of the nucleic acid to prevent degradation by nucleases such as exonuclease, RNase, etc. Such capping groups include, but are not limited to, groups for protecting hydroxyl known in the art, including glycols such as polyethylene glycol, tetraethylene glycol, etc.

The inhibitory activity of the antisense nucleic acid can be examined using the transformant of the invention, the in vivo or in vitro gene expression system of the invention, or the in vivo or in vitro translation system of the protein of the invention. These nucleic acids can be applied to cells by a variety of publicly known methods.

The DNA encoding the partial peptide of the invention may be any DNA which contains the base sequence encoding the partial peptide of the invention described above. The DNA may be derived from any of genomic DNAs, genomic DNA library, cDNAs derived from the cells and tissues described above, cDNA library derived from the cells and tissues described above and synthetic DNA.

The DNA encoding the partial peptide of the invention may be, for example, a DNA having a partial base sequence of the DNA having the base sequence represented by SEQ ID NO: 2, or a DNA having a partial base sequence of a DNA hybridizable to the DNA having the base sequence represented by SEQ ID NO: 2 under high stringent conditions and encoding the protein which has the activity substantially equivalent to the activity of the protein having the amino acid sequence represented by SEQ ID NO: 1.

Examples of the DNA that is hybridizable to the DNA having the base sequence represented by SEQ ID NO: 2 under high stringent conditions include DNAs having a base sequence with at least about 97% homology, preferably at least about 98% homology, and more preferably at least about 99% homology to the base sequence represented by SEQ ID NO: 2.

Methods for the hybridization and the high stringent conditions that can be used are the same as described above.

For cloning of the DNA that completely encodes the protein of the invention or the partial peptide of the invention (hereinafter sometimes collectively referred to as the protein of the invention in the following description of cloning and expression of the DNA encoding these proteins or the like), the DNA may be either amplified by publicly known PCR using synthetic DNA primers containing a part of the base sequence encoding the protein of the invention, or the DNA inserted into an appropriate vector can be selected by hybridization with a labeled DNA fragment or synthetic DNA that encodes a part or the entire region of the protein of the invention. The hybridization can be carried out, for example, according to the method described in Molecular Cloning, 2nd (J. Sambrook et al., Cold Spring Harbor Lab. Press, 1989). The hybridization may also be performed using commercially available library in accordance with the protocol described in the attached instructions.

Conversion of the base sequence of DNA can be effected by publicly known methods such as the ODA-LAPCR method, the Gapped duplex method or the Kunkel method, or modifications thereof, by using publicly known kits available as Mutan™-super Express Km (TaKaRa Shuzo Co., Ltd.) or Mutan™-K (TaKaRa Shuzo Co., Ltd.), etc.

The cloned DNA encoding the protein of the invention can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the invention can be manufactured, for example, by (a) excising the desired DNA fragment from the DNA encoding the protein of the invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, HIV/LTR promoter, CMV promoter, HSV-TK promoter, etc.

Among them, CMV (cytomegalovirus) promoter or SRα promoter is preferably used. When bacteria of the genus Escherichia are used as the host, preferred examples of the promoter include trp promoter, lac promoter, recA promoter, λPL promoter, lpp promoter, T7 promoter, etc. When bacteria of the genus Bacillus are used as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin promoter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo, G418 resistance), etc. In particular, when dhfr gene is used as the selection marker together with dhfr gene-deficient Chinese hamster cells, selection can also be made on thymidine-free media.

If necessary, a signal sequence that matches with a host is added to the N-terminus of the protein of the invention. Examples of the signal sequence that can be used are Pho A signal sequence, OmpA signal sequence, etc. in case of using bacteria of the genus Escherichia as the host; α-amylase signal sequence, subtilisin signal sequence, etc. in case of using bacteria of the genus Bacillus as the host; MFα signal sequence, SUC2 signal sequence, etc. in case of using yeast as the host; and insulin signal sequence, α-interferon signal sequence, antibody molecule signal sequence, etc. in case of using animal cells as the host, respectively.

Using the vector bearing the DNA encoding the protein of the invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)), JA221 (Journal of Molecular Biology, 120, 517 (1978)], HB101 [Journal of Molecular Biology, 41, 459 (1969)), C600 (Genetics, 39, 440 (1954)], etc.

Examples of the bacteria belonging to the genus Bacillus include Bacillus subtilis MI114 [Gene, 24, 255 (1983)], 207-21 (Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711) and Sf21 cell (both cells are described in Vaughn, J. L. et al., In Vivo, 13, 213-217 (1977).

As the insect, for example, a larva of Bombyx mori can be used (Maeda et al., Nature, 315, 592, 1985).

Examples of animal cells include monkey cell COS-7, Vero, Chinese hamster cell CHO (hereinafter referred to as CHO cell), dhfr gene-deficient Chinese hamster cell CHO (hereinafter simply referred to as CHO(dhfr⁻) cell), mouse L cell, mouse AtT-20, mouse myeloma cell, rat GH 3, human FL cell, H9c2 cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991) or Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector containing the DNA encoding the protein can be obtained.

When the host is a bacterium belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extracts, vitamins, growth-stimulating factors etc. may also be added to the medium. Preferably, pH of the medium is adjusted to about 5 to about 8.

A preferred example of the medium for culturing bacteria belonging to the genus Escherichia is M9 medium supplemented with glucose and casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to activate the promoter efficiently.

When a bacterium belonging to the genus Escherichia is used as the host, the transformant is usually cultivated at about 15 to 43°C for about 3 to 24 hours. If necessary, the culture may be aerated or agitated.

When a bacterium belonging to the genus Bacillus is used as the host, the transformant is cultivated generally at about 30 to 40°C for about 6 to 24 hours. If necessary, the culture can be aerated or agitated.

When yeast is used as the host, the transformant is cultivated, for example, in Burkholder's minimal medium (Bostian, K. L. et al., Proc. Natl. Acad. Sci. U.S.A., 77, 4505, 1980) or in SD medium supplemented with 0.5% Casamino acids (Bitter, G. A. et al., Proc. Natl. Acad. Sci. U.S.A., 81, 5330, 1984). Preferably, pH of the medium is adjusted to about 5 to about 8. In general, the transformant is cultivated at about 20 to 35°C for about 24 to 72 hours. If necessary, the culture can be aerated or agitated.

When an insect cell or insect is used as the host, the transformant is cultivated in, for example, Grace's Insect Medium (Grace, T. C. C., Nature, 195, 788 (1962)) to which an appropriate additive such as 10 % inactivated bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

When an animal cell is employed as the host, the transformant is cultivated in, for example, MEM medium (Science, 122, 501 (1952)], DMEM medium (Virology, 8, 396 (1959)], RPMI 1640 medium (The Journal of the American Medical Association, 199, 519 (1967)), or 199 medium (Proceeding of the Society for the Biological Medicine, 73, 1 (1950)), which contains about 5% to about 20% fetal bovine serum. Preferably, pH of the medium is adjusted to about 6 to about 8. The transformant is usually cultivated at about 30 to 40°C for about 15 to 60 hours and, if necessary, the culture can be aerated or agitated.

As described above, the protein of the invention can be produced inside or outside of the transformant cell.

The protein of the invention can be separated and purified from the culture described above by the following procedures.

When the protein of the invention is extracted from the cultured bacteria or cells, after cultivation the bacteria or cells are collected by a publicly known method and suspended in an appropriate buffer. The bacteria or cells are then disrupted by publicly known methods such as ultrasonication, a treatment with lysozyme and/or freeze-thaw cycling, followed by centrifugation, filtration, etc. Thus, the crude proteinous extract can be obtained. The buffer used for the procedures may contain a protein modifier such as urea or guanidine hydrochloride, or a surfactant such as Triton X-100™, etc. When the protein of the invention is secreted into the culture broth, after completion of the cultivation the supernatant can be separated from the bacteria or cells to collect the supernatant by publicly known methods.

The protein of the invention contained in the supernatant or the extract thus obtained can be purified by an appropriate combination of the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reversed phase high performance liquid chromatography, etc.; a method utilizing difference in isoelectric point such as isoelectrofocusing electrophoresis; and the like.

When the protein of the invention thus obtained is in a free form, it can be converted into a salt form by publicly known methods or modifications thereof. On the other hand, when the protein is obtained in a salt form, it can be converted into the free form or a different salt by publicly known methods or modifications thereof.

The protein of the invention produced by the recombinant can be treated, before or after the purification, with an appropriate protein-modifying enzyme so that the protein can be appropriately modified or partially deleted. Examples of the protein-modifying enzyme include trypsin, chymotrypsin, arginyl endopeptidase, protein kinase, glycosidase and the like.

The presence of the thus produced protein of the invention can be determined by an enzyme immunoassay using a specific antibody, western blotting method, or the like.

Antibodies to the protein of the invention, its partial peptide, or salts thereof, may be any polyclonal antibodies or monoclonal antibodies, as long as they are capable of recognizing the protein of the invention, its partial peptide, or salts thereof.

The antibodies to the protein of the invention, its partial peptide, or salts thereof (hereinafter in the description of antibodies, these terms are occasionally referred to simply as the protein of the invention) may be manufactured by publicly known methods for manufacturing antibodies or antisera, using as an antigen the protein of the invention.

### [Preparation of monoclonal antibody]

### (a) Preparation of monoclonal antibody-producing cells

The protein of the invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and two to ten times in total. Examples of the applicable warm-blooded animals are monkeys, rabbits, dogs, guinea pigs, mice, rats, sheep, goats and chickens, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature, 256, 495 (1975)]. Examples of the fusion promoter are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by culturing at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein (protein) as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase; etc.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at 37°C, for about 5 days to about 3 weeks, preferably 1 to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by publicly known methods, such as separation and purification of immunoglobulins (for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G, and dissociating the binding to obtain the antibody].

### [Preparation of polyclonal antibody]

The polyclonal antibody of the invention can be manufactured by publicly known methods or modifications thereof. For example, a warm-blooded animal is immunized with an immunogen (protein of the invention as an antigen) per se, or a complex of immunogen and a carrier protein is formed and a warm-blooded animal is immunized with the complex in a manner similar to the method described above for the manufacture of monoclonal antibodies. The product containing the antibody to the protein of the invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide-activated ester, activated ester reagents containing thiol group or dithiopyridyl group, and others are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks about 3 to 10 times in total.

The polyclonal antibody can be collected from the blood, ascites, etc., preferably from the blood of warm-blooded animals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described above.

An antisense nucleotide having a base sequence complementary or substantially complementary to the DNA encoding the protein or the partial peptide of the present invention (hereinafter, the DNA may be referred to as the DNA of the present invention) may be any antisense nucleotide having a base sequence complementary or substantially complementary to the DNA of the present invention, or a partial base sequence thereof, and having an activity to inhibit the expression of the DNA; an antisense DNA is preferred.

The base sequence substantially complementary to the DNA of the invention includes base sequences having about 97% or higher, preferably about 98% or higher, and more preferably about 99% or higher homology to the whole or a part of the base sequence complementary to the DNA of the invention (i.e. a complementary strand of the DNA of the invention). Particularly, a preferable antisense nucleotide is one having about 97% or higher, preferably about 98% or higher, and more preferably about 99% or higher homology to the complementary strand of the partial base sequence (e.g. the base sequence around an initiation codon) encoding the N-terminal region of the protein of the invention within the whole base sequence of the complementary strand of the DNA of the invention.

The antisense nucleotide may consist of usually about 10 to 40 and preferably 15 to 30 bases.

To prevent the degradation by hydrolase such as nuclease, phosphate residues (phosphates) of respective nucleotides constituting the antisense nucleotide may be substituted with chemically modified phosphate residues such as phosphorothioate, methyl phosphonate, phosphorodithionate, etc. These antisense nucleotides can be manufactured by using a publicly known DNA synthesizer.

Hereinafter the utilities of the protein of the invention or its partial peptide, or salts thereof (hereinafter sometimes collectively referred to as the protein of the invention); the DNA encoding the protein of the invention or its partial peptide (hereinafter sometimes collectively referred to as the DNA of the invention), and the antibody to the protein of the invention or its partial peptide, or salts thereof (hereinafter sometimes collectively referred to as the antibody of the invention); and the antisense nucleotide to the DNA of the present invention (hereinafter occasionally referred to as the antisense nucleotide of the present invention).

The protein of the invention expresses in a heart increasingly during a transition period to heart failure after cardiac infarction, and hence can be used as a disease marker. It is useful as a marker for making an early diagnosis of diseases characterized by the cardiac hypofunction (e.g. heart diseases such as heart failure after cardiac infarction; angina pectoris; myocardosis; heart failure derived from diseases such as angina pectoris, myocardosis, etc.), for determining severity of the diseases, and for predicting progress of the diseases.

Pharmaceuticals containing an antisense nucleotide to the gene encoding the protein of the invention, a compound or a salt thereof regulating the activity of the protein of the invention, or an antibody to the protein of the invention are useful as remedies and/or preventives for diseases characterized by the cardiac hypofunction (e.g. heart diseases such as heart failure after cardiac infarction; angina pectoris; myocardosis; heart failure derived from diseases such as angina pectoris, myocardosis, etc.).

### [1] Screening of compounds as candidates of pharmaceuticals for diseases

The protein of the invention expresses increasingly in accordance with lowering of the cardiac function after cardiac infarction and hence, the compound regulating the activity of the protein of the invention or a salt thereof can be used as the remedies and/or preventives of diseases characterized by the cardiac hypofunction (e.g. heart diseases such as heart failure after cardiac infarction; angina pectoris; myocardosis; heart failure derived from diseases such as angina pectoris, myocardosis, etc.).

Therefore, the protein of the invention is useful as a reagent for screening of the compound or its salt regulating the activity of the protein of the invention.

Thus, the present invention provides:
(1) a screening method for a compound or a salt thereof regulating an activity of the protein of the present invention, which comprises using the protein of the invention;
   specifically, for example,
(2) the screening method for a compound or a salt thereof regulating the activity of the protein of the invention, which comprises comparing (i) the case where a stretching stimulation is imposed on a cell having an ability of producing the protein of the invention preferably under a low oxygen condition, with (ii) the case where a stretching stimulation is imposed on a cell having an ability of producing the protein of the invention in the presence of a test compound preferably under a low oxygen condition.

More specifically, the above screening method is characterized in that, for example, an expression level of the gene of the protein of the invention is measured and compared in cases of (i) and (ii).

The activity of the protein of the invention includes, for example, the activity of enhancing the cardiac hypofunction, which is caused by compensation failure, and the activity of suppressing the excessive expression of compensation mechanisms.

The aforementioned low oxygen condition means the condition of a 20% or lower oxygen concentration such as 2% (Nature 394: 485-490, 1998). The stretching stimulation means a mechanical stimulation to a myocardial cell, generated by stretching the stretchable silicon film, on which a myocardial cell is cultured (J.B.C. 271: 33592-33597, 1996; Circulation 89: 2204-2211, 1994; J.B.C. 271: 3221-3228, 1996).

In addition, the invention provides:
(3) the screening method for a compound or a salt thereof regulating the activity of the protein of the invention, which comprises comparing (iii) the case where a cell having an ability of producing the protein of the invention or a cell, into which the cDNA encoding the protein of the invention is transfected, is cultured under the lethal condition (for example, cultured without serum or with an added anticancer agent such as adriamycin having a relatively strong toxicity to a myocardial cell), with (iv) the case where a cell having an ability of producing the protein of the invention or a cell, into which the cDNA encoding the protein of the invention is transfected, is cultured in the presence of a test compound under the lethal condition (for example, cultured without serum or with an added anticancer agent such as adriamycin having a relatively strong toxicity to a myocardial cell).

In the above-described screening method, for example, a cell-protecting effect or the expression amount of the gene encoding the protein of the invention is measured by a publicly known method and compared in cases of (iii) and (iv).

The cell-protecting effect can be expressed by a ratio of activated or surviving myocardial cells. Specifically, the ratio can be determined by such methods well used in general as the MTT (3-(4,5-Dimethyl-2-thiazolyl)-2,5-diphenyl-2H-tetrazolium) method that can measure respiratory activity, trypan blue staining method, or the TUNNEL Staining method (Terminal deoxytransferase-mediated d UTP-X nick end labeling, Cell 97: 189-198, 1999).

It can be expected that a proper increase in the gene expression at cell death or cell damage generates the cell-protecting effect. It is presumed that an excessive increase in the gene expression induces an excessive activation of the cells resulting in fatigue of the cells. Therefore, controlling properly the amount of gene expression is considered important. For example, in case where cell damage may have occurred through enhancement of the gene expression as during the period of cardiac failure, an inhibitor (the compound inhibiting the activity of the protein of the invention or a salt thereof) may be administered and, for example, in case where cell damage may have occurred through lowering of the gene expression as in an acute stage after cardiac infarction or in a stage of insufficient expression of compensation mechanisms, an accelerator (the compound accelerating the activity of the protein of the invention or a salt thereof) may be administered.

It is presumed that the protein of the invention activates GTPase activity of a G-protein. It has been known that GTPase activity of a G-protein accelerates dissociation of subunits of the G-protein to suppress signal transmission through the G-protein. Concerning heart failure, it is presumed that abnormality of the G-protein signal transmission mechanism, e.g. desensitization of β-receptor, makes condition of heart failure become worse, and thus it is also presumed that enhancement of expression of the DNA which encodes the protein or partial peptide of the invention (hereafter occasionally referred to as the DNA of the invention) involves aggravation of heart failure.

Therefore, the screening for the compound regulating the activity of the protein of the invention or a salt thereof can be carried out using the cell having the ability of producing the protein of the invention or the cell, into which the cDNA encoding the protein of the invention is transfected, and using as an index the production of the signal transmission substance (e.g. cAMP, calcium, etc.), which is mediated by the G protein stimulated by a ligand.

Specifically, the invention provides:

A screening method for a compound or a salt thereof regulating expression of the gene encoding the protein of the invention, characterized by comparing (v) the case where the cell having then ability of producing the protein of the invention or the cell, into which the cDNA encoding the protein of the invention is transfected, is cultured under stimulation by a ligand to a G-protein-coupling receptor, such as β-stimulation, with (vi) the case where the cell having then ability of producing the protein of the invention or the cell, into which the cDNA encoding the protein of the invention is transfected, is cultured in the presence of a test compound under stimulation by a ligand to a G-protein-coupling receptor, such as β-stimulation.

In the screening method as described above, for example, a suppressing effect or enhancing effect of signal transmission is measured by a publicly known method and compared in cases of (v) and (vi).

Cells preferably used may be a primary cultured myocardial cell of a rat or a mouse, H9c2 cell line derived from a rat ventricle, etc.

For example, for a patient in a compensation period for heart failure, in which the gene expression is lowered, the compound or a salt thereof enhancing expression of the gene of the invention is preferably used. In a heart failure period, in which the gene expression is excessive, the compound or a salt thereof suppressing expression of the gene of the invention is preferably used.

The test compound includes, e.g., peptides, proteins, peptide-like compounds (saccharides, lipids, etc.) derived from an organism, synthetic compounds, microorganism cultures, cell extracts, plant extracts, animal tissue extracts, etc. and these compounds may be novel or publicly known.

For carrying out the above-described screening methods, the cell having the ability of producing the protein of the invention is cultured in a culture medium suitable for the screening. Any medium can be used unless it has any influence on the gene expression for the protein of the invention.

The cell having the ability of producing the protein of the invention may be, for example, the primary cultured myocardial cell having intrinsically the ability of producing the protein of the invention or a host cell transformed by a vector comprising the DNA encoding the protein of the invention (transformant) as described above. Preferably usable hosts are such animal cells as H9c2 cells (ATCC No. CRL-1446), etc. For the screening, a preferably usable transformant is one in which the protein of the invention has been expressed in its cytoplasm by culturing according to the above-described method.

The amount of gene expression can be measured by publicly known methods such as the northern blotting method, the reverse transcription-polymerase chain reaction (RT-PCR,) or the real time PCR analysis system (ABI, TaqMan polymerase chain reaction) or similar methods.

For example, a test compound inhibiting or enhancing the amount of the gene expression in the case (ii) by about 20% or more, preferably about 30% or more, and more preferably about 50% or more, as compared with the case (i) can be selected as the compound inhibiting or enhancing the activity of the protein of the invention.

A recovery effect on the cardiac function can be expected when administering the compound or its salt inhibiting the activity of the protein of the invention (inhibitor), which is selected by the above-described screening methods, in an end stage of heart failure where the expression of the DNA (gene) of the invention is enhanced. On the other hand, a heart protective effect can be expected when administering the compound or its salt enhancing the activity of the protein of the invention (enhancer), which is selected by the above-described screening methods, in a chronic stage of heart failure where the expression is lowered, because the compound can suppress the excessive expression of compensation mechanisms to protect myocardial cells.

The screening kit of the invention comprises the protein used in the invention, its partial peptide or a salt thereof, or the cell having the ability of producing the protein used in the invention or its partial peptide.

The compound or its salt (the compound enhancing or inhibiting the activity of the protein of the invention), which is obtained by the screening method or the screening kit of the invention, is one selected from the above described test compounds such as peptides, proteins, non-peptide compounds (e.g. saccharides, lipids, etc.) derived from an organism, synthetic compounds, microorganism cultures, fermentation products, cell extracts, plant extracts, animal tissue extracts, blood plasma, and is one which regulates (enhances or inhibits) the activity of the protein of the invention (the activity of enhancing cardiac hypofunction and the like).

The salts of the compound used are the same kinds as the above-described salts of the protein of the invention.

The compounds regulating (enhancing or inhibiting) the activity of the protein of the invention, or salts thereof are useful as pharmaceuticals such as remedies or preventives for diseases characterized by the cardiac hypofunction (e.g. heart diseases such as heart failure after cardiac infarction; angina pectoris; myocardosis; heart failure derived from diseases such as angina pectoris, myocardosis, etc.).

When using the compounds, which are obtained by the screening method or the screening kit of the invention, or salts thereof as the above-described remedies or preventives, they can be formulated in a conventional manner. For example, forms of tablets, capsules, elixir, microcapsules, sterile solution, suspension, etc. are possible.

Such preparations obtained are safe and low toxic and thus can be administered orally or parenterally to, for example, a human or other warm-blooded animals (e.g. mouse, rat, rabbit, sheep, swine, bovine, horse, fowl, cat, dog, monkey, chimpanzee, etc.).

The dose of the compound or its salt varies depending on its effect, a target disease, a subject to be administered, a route for administration, etc. For example, when administering orally the compound or its salt regulating the activity of the protein of the invention to an adult (60 kg body weight) for the treatment of heart failure, the daily dose is normally about 0.1 to 100 mg, preferably about 1.0 to 50 mg, and more preferably about 1.0 to 20 mg of the compound. In parenteral administration, the single dose of the compound or its salt also varies depending on a subject to be administered, a target disease, etc. For example, when administering the compound or its salt regulating the activity of the protein of the invention to an adult (60 kg body weight) for the treatment of heart failure in an injectable form, it is advantageous to inject intravenously the compound or its salt at a daily dose of about 0.01 to 30 mg, preferably about 0.1 to 20 mg, and more preferably about 0.1 to 10 mg. For other animal species, the corresponding dose as converted per 60 kg body weight can be administered.

### [2] Quantification of the protein of the invention, its partial peptide or a salt thereof

The antibody to the protein of the invention (hereinafter sometimes simply referred to as the antibody of the invention) is capable of specifically recognizing the protein of the invention and thus, can be used for a quantification of the protein of the invention in a test liquid sample, in particular, for the quantification by sandwich immunoassay.

Thus, the present invention provides:
(i) a method for quantification of the protein of the invention in a test liquid sample, which comprises competitively reacting the antibody of the invention with the test liquid sample and a labeled form of the protein of the invention, and measuring the ratio of the labeled protein of the invention; and,
(ii) a method for quantification of the protein of the invention in a test liquid sample, which comprises reacting the test liquid sample simultaneously or sequentially with the antibody of the invention immobilized on a carrier and a labeled form of another antibody of the invention, and then measuring the activity of the label on the immobilizing carrier.

In the quantification method (ii) described above, it is preferred that one antibody is capable of recognizing the N-terminal region of the protein of the invention, while another antibody is capable of reacting the C-terminal region of the protein of the invention.

The monoclonal antibody to the protein of the invention (hereinafter sometimes referred to as the monoclonal antibody of the invention) can be used to quantify the protein of the invention. Moreover, the protein of the invention can be detected by a tissue staining method as well. For these purposes, the antibody molecule per se may be used or F(ab')₂, Fab' or Fab fractions of the antibody molecule may also be used.

There is no particular limitation for the type of quantification method using the antibody to the protein of the invention, and any assay methods can be used whereby the amount of antibody, antigen, or antibody-antigen complex corresponding to the amount of antigen (e.g., the amount of the protein) in the test liquid can be detected by chemical or physical means and the amount of the antigen can be calculated from a standard curve prepared from standard solutions containing known amounts of the antigen. Advantageously used are, for example, nephrometry, competitive method, immunometric method and sandwich method. In terms of sensitivity and specificity, the sandwich method, which will be described later, is particularly preferred.

Examples of the labeling agent used in the assay method using the labeled substance are radioisotopes, enzymes, fluorescent substances and luminescent substances, etc. Examples of the radioisotope are [¹²⁵I], [¹³¹I], [³H], [¹⁴C], etc. Preferred examples of the enzyme are ones that are stable and have a high specific activity, and include β-galactosidase, β-glucosidase, alkaline phosphatase, peroxidase, malate dehydrogenase, etc. Examples of the fluorescent substance are fluorescamine, fluorescein isothiocyanate, etc. Examples of the luminescent substance are luminol, luminol derivatives, luciferin, lucigenin, etc. Furthermore, the biotin-avidin system may also be used for binding of an antibody or antigen to a labeling agent.

For the immobilizatio of antigens or antibodies, physical adsorption may be used. Alternatively, chemical binding that is conventionally used for immobilization of proteins or enzymes may be used as well. Examples of the carrier include insoluble polysaccharides such as agarose, dextran and cellulose; synthetic resins such as polystyrene, polyacrylamide, silicone; or glass, etc.

In the sandwich method, a test liquid sample is reacted with an immobilized monoclonal antibody of the invention (primary reaction), then reacted with another labeled monoclonal antibody of the invention (secondary reaction) and the activity of the label on the immobilizing carrier is assayed, whereby the amount of the protein of the invention in the test liquid sample can be quantified. The primary and secondary reactions may be carried out in a reversed order, simultaneously or sequentially with an interval. The type of the labeling agent and the method for immobilization may be the same as those described above. In the immunoassay by the sandwich method, it is not always necessary that one type of the antibody is used for the immobilized or labeled antibody, but a mixture of two or more antibodies may also be used for the purpose of improving the measurement sensitivity, etc.

In the quantification of the protein of the invention by the sandwich method, it is preferred that the monoclonal antibodies of the invention used for the primary and secondary reactions have different binding sites on the protein of the invention, respectively. Thus, in respect to the antibodies used in the primary and secondary reactions, for example, when the antibody used in the secondary reaction recognizes the C-terminal region of the protein of the invention, the antibody used in the primary reaction preferably recognize a region other than the C-terminal region, for example, the N-terminal region.

The monoclonal antibody of the invention may be used in an assay system other than the sandwich method, such as a competitive method, an immunometric method, a nephrometry, etc.

In the competitive method, an antigen in a test liquid sample and a labeled antigen are competitively reacted with an antibody, then the unreacted labeled antigen (F) and the labeled antigen bound to the antibody (B) are separated (i.e. B/F separation), and the amount of the label present in either B or F is measured to determine the amount of the antigen in the test liquid sample. In the reactions for such a method, there are a liquid phase method in which a soluble antibody is used as the antibody and the B/F separation is effected by polyethylene glycol and a second antibody to the antibody, and a solid phase method in which an immobilized antibody is used as the first antibody or a soluble antibody is used as the first antibody while an immobilized antibody is used as the second antibody.

In the immunometric method, an antigen in a test liquid sample and an immobilized antigen are competitively reacted with a given amount of the labeled antibody, followed by separating the solid phase from the liquid phase; or an antigen in a test liquid sample and an excess amount of the labeled antibody are reacted, then an immobilized antigen is added to bind the unreacted labeled antibody to the solid phase and the solid phase is separated from the liquid phase. Thereafter, the amount of the label in either of the phases is measured to determine the antigen amount in the test liquid sample.

In the nephrometry, the amount of insoluble sediment, which is produced as a result of the antigen-antibody reaction in a gel or in a solution, is measured. Even when the amount of an antigen in a test liquid sample is small and only a small amount of the sediment is obtained, a laser nephrometry utilizing laser scattering can be suitably used.

For applying these immunological methods to the quantification method of the invention, any special conditions or procedures are not required. A system for quantifying the protein of the invention may be constructed according to the combination of the usual technical consideration in the art and the conventional conditions and procedures. For the details of these general technical means, reference can be made to any reviews and textbooks.

For example, see Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies))(all published by Academic Press).

As described above, the protein of the invention can be quantified with high sensitivity, using the antibody of the invention.

Furthermore, when an increased level of the protein of the invention is detected in a subject by quantifying the protein level using the antibody of the invention, the subject can be diagnosed as highly likely to suffer from, at that time or in the future, diseases characterized by the cardiac hypofunction (e.g. heart diseases such as heart failure after cardiac infarction; angina pectoris; myocardosis; heart failure derived from diseases such as angina pectoris, myocardosis, etc.).

The antibody of the present invention can be employed for detecting the protein of the invention that may be present in a test sample such as a body fluid, a tissue, etc. The antibody can also be used for preparation of an antibody column for purification of the protein of the invention, detection of the protein of the invention in fractions upon purification, and analysis of the behavior of the protein of the invention in the cells under investigation.

### [3] Genetic diagnosis agents

By using the DNA of the invention, e.g., as a probe, an abnormality of the DNA or mRNA encoding the protein of the invention or its partial peptide in human or any other warm-blooded animals (e.g., rat, mouse, guinea pig, rabbit, fowl, sheep, swine, bovine, horse, cat, dog, monkey, chimpanzee, etc.) (gene abnormality) can be detected. Therefore, the DNA of the invention is useful as a genetic diagnosis agent for detecting the damage, mutation, decreased expression, or increased expression or overexpression of said DNA or mRNA.

The genetic diagnosis described above using the DNA of the invention can be performed by, for example, the publicly known northern hybridization assay or the PCR-SSCP assay (Genomics, 5, 874-879 (1989); Proceedings of the National Academy of Sciences of the United States of America, 86, 2766-2770 (1989)), etc.

For example, when an increased expression of the DNA is detected in a subject by the northern hybridization assay or when a mutation of the DNA is detected by the PCR-SSCP assay, the subject can be diagnosed as highly likely to suffer from diseases such as diseases accompanied with cardiac hypofunction, etc.

### [4] Pharmaceuticals containing the antisense nucleotide

The antisense nucleotide of the invention capable of binding complementally to the DNA of the present invention and of suppressing the expression of the DNA shows low toxicity and can regulate (inhibit) the in vivo functions of the protein or the DNA of the present invention (e.g. the activity of enhancing cardiac hypofunction) and hence, can be used as, for example, the prophylactic and therapeutic agent for diseases such as diseases characterized by the cardiac hypofunction (e.g. heart diseases such as heart failure after cardiac infarction; angina pectoris; myocardosis; heart failure derived from diseases such as angina pectoris, myocardosis, etc.).

When used as the prophylactic and therapeutic agent as described above, the aforementioned antisense nucleotide can be formulated for administration according to the publicly known method.

For example, when used, the antisense nucleotide can be orally or parenterally administered to a human or any other warm-blooded animals (e.g., rat, mouse, rabbit, sheep, swine, bovine, horse, fowl, feline, canine, monkey, chimpanzee, etc.) in a conventional manner, alone or after inserted into a proper vector such as retrovirus vector, adenovirus vector, adenovirus-associated virus vector, etc. The antisense DNA can be administered using a gene gun or such a catheter as a hydrogel catheter, as it is, or as formulated with a physiologically acceptable carrier such as an auxiliary material for uptake enhancement.

The dose of the antisense nucleotide varies depending on target disease, subject to be administered, route for administration, etc. For example, when the antisense nucleotide of the invention is orally administered for the treatment of heart failure, it is administered to the adult (60 kg body weight) in a daily dose of about 0.1 to 100 mg.

Further, the antisense nucleotide can be used as a diagnostic oligonucleotide probe to examine the presence or expression profile of the DNA of the present invention in a tissue or a cell.

The present invention further provides:
(1) a double stranded RNA comprising a part of the RNA encoding the protein of the present invention;
(2) a pharmaceutical comprising the double stranded RNA;
(3) a ribozyme comprising a part of the RNA encoding the protein of the present invention; and
(4) a pharmaceutical comprising the ribozyme.

The double stranded RNA and ribozyme, like the antisense polynucleotide, can suppress the expression of the polynucleotide (e.g., DNA) of the invention can inhibit the in vivo functions (e.g., activity of enhancing cardiac hypofunction) of the peptide or the polynucleotide of the present invention and hence can be used as, for example, the prophylactic and therapeutic agent for diseases characterized by the cardiac hypofunction (e.g. heart diseases such as heart failure after cardiac infarction; angina pectoris; myocardosis; heart failure derived from diseases such as angina pectoris, myocardosis, etc.).

The double stranded RNA can be designed on the basis of the sequence of the polynucleotide of the present invention according to the publicly known method (e.g., Nature 411: 494, 2001) and then manufactured.

The ribozyme can be designed on the basis of the sequence of the polynucleotide of the present invention according to the publicly known method (e.g.,Trends in Mol. Med. 7: 221. 2001) and then manufactured. For example, it can be manufactured by connecting a part of the RNA encoding the peptide of the present invention to a publicly known ribozyme. Such a part of the RNA encoding the peptide of the present invention includes the part (RNA fragment) near a cleavage site on the RNA of the present invention, which is cleavable by the publicly known ribozyme.

When used as the above-described preventives or remedies, the aforementioned double stranded RNA or ribozyme can be formulated for administration in a similar manner to that for the antisense polynucleotide.

### [5] Pharmaceuticals containing the antibody of the invention

The antibody of the invention having an effect to neutralize the activity of the protein of the invention can be used as the prophylactic and therapeutic agent for diseases characterized by the cardiac hypofunction (e.g. heart diseases such as heart failure after cardiac infarction; angina pectoris; myocardosis; heart failure derived from diseases such as angina pectoris, myocardosis, etc.).

The prophylactic and therapeutic agent containing the antibody of the invention for the aforementioned diseases are low toxic and can be orally or parenterally administered to a human and other warm-blooded animals (e.g., mouse, rat, rabbit, sheep, swine, bovine, horses, fowl, feline, canine, monkey, chimpanzee, etc.) as an intact solution or a pharmaceutical composition in a proper form.

The dose varies depending on a subject to be administered, a target disease, a symptom, a route for administration, etc. When used for the purpose of prevention or treatment for, e.g., heart failure of an adult, it is preferable that the antibody of the invention is intravenously administered normally in a daily dose of about 0.01 mg to about 20 mg/ kg body weight, preferably about 0.1 to about 10 mg/ kg body weight, and more preferably about 0.1 to about 5 mg/ kg body weight, once to 5 times a day and preferably once to 3 times a day. A dose similar to those given above can be administered in another parenteral administration and an oral administration. When a symptom is very severe, the dose may be increased depending on the symptom.

The antibody of the invention can be administered as it is or as a proper pharmaceutical composition. The pharmaceutical composition used for the above-described administration contains the antibody of the invention or its salt with a physiologically acceptable carrier, a diluting agent, or a vehicle. Such a composition is provided in an orally or parentally suitable drug form.

Thus, for example, the composition for oral administration is exemplified by a solid or liquid drug form, specifically a tablet (including sugar-coated and film-coated tables,) pill, granule, powder, capsule (including soft capsule,) syrup, emulsion, suspension, etc. Such a composition is manufactured by a publicly known method and contains a carrier, diluting agent, or vehicle generally used in pharmaceutical field. For example, as the carrier and vehicle for the tablet, lactose, starch, sucrose, magnesium stearate, etc. are used.

The composition used for parenteral administration includes, e.g., an injection, a suppository, etc. and the injection includes forms such as intravenous injection, subcutaneous injection, endodermic injection, muscular injection, drop injection, etc. Such injections may be prepared by dissolving, suspending, or emulsifying the antibody or its salt as described above in sterile aqueous or oily solution generally used for an injection following the publicly known method. The aqueous solution used for an injection includes, for example, a physiological saline, an isotonic solution containing glucose or any other auxiliary agent and may be used in combination with a proper dissolving aid such as alcohol (e.g. ethanol), polyalcohol (e.g. propylene glycol, polyethylene glycol), nonionic surfactant (e.g. polysorbate 80, HCO-50 (polyoxyethylene (50 mol) adduct of hydrogenated castor oil)). The oily solution includes, for example, sesame oil, soybean oil, etc., and may be used in combination with benzyl benzoate, benzyl alcohol, etc. as the dissolving aid. As a rule, the injection prepared is filled in a proper ampoule. The suppository used for administration into rectum is prepared by compounding the antibody or its salt as described above with a normal base material for a suppository.

It is advantageous to prepare the above-described pharmaceutical composition for an oral or parenteral use in a dosage unit form suitable for the dosage of the active component. The dosage unit form is exemplified by the table, pill, capsule, injection (ampoule,) suppository, etc. It is preferable that the dosage unit form normally contains 5 to 500 mg; particularly 5 to 10 mg for the injection, and 10 to 250 mg in other forms.

These compositions as described above may contain other active components unless there generates any undesirable interaction thereof with the aforementioned antibody in a mixture.

### [6] Pharmaceuticals containing the protein of the invention

The pharmaceutical containing the protein of the invention can be used as a vaccine for production of the antibody of the invention. The vaccine can be manufactured by using the protein of the invention according to a publicly known method.

### [7] Pharmaceuticals containing the DNA of the invention

The pharmaceutical containing the DNA of the invention can be used for gene therapy of heart failure. It is presumed that a function of the DNA of the invention is a part of the biological defence system to suppress the excessive expression of compensation mechanisms. For example, it has been known that an adrenalin receptor binds to adrenalin discharged from the sympathetic nerve to express a cardiotonic effect. It has been also known that angiotensin II receptor binds to angiotensin II to participate in heart remodeling including megalocardia. The compensation mechanisms include activation of these mechanisms. However, it is presumed that an excessive action of adrenalin may accelerate heart fatigue and the heart remodeling itself caused by the excessive action of angiotensin II is one of aggravating actions of heart failure (Naika (Internal medicine) 79: 2-20, 1997). It is known that these said receptors are G protein-coupled receptors and antagonists of the receptors are effective on heart failure. The function of the protein of the present invention is to suppress signal transmission from these receptors and thus, it is presumed that the DNA of the invention is also useful for the gene therapy of heart failure.

### [8] Preparation of animals having the DNA of the present invention

Using the DNA of the present invention, transgenic animals that express the protein of the present invention can be prepared. Examples of the animals include mammals (e.g., rats, mice, rabbits, sheep, swine, bovine, cats, dogs, monkeys, etc.) or the like (hereinafter sometimes referred to as animals), with particularly preferred being mice, rabbits, etc.

When transfecting the DNA of the present invention into a target animal, it is generally advantageous to use a gene construct in which the DNA is ligated downstream of a promoter capable of expressing the DNA in an animal cell. For example, when transfecting the rabbit-derived DNA of the present invention, the gene construct, in which the DNA of the present invention, derived from animals, with high homology with the aforementioned DNA is ligated downstream of various promoters that can express the DNA of the present invention in an animal cell, is microinjected to, e.g., a rabbit fertilized egg. Thus, the DNA-transfected animal capable of producing a high level of the protein of the present invention can be prepared. Examples of the promoter that can be used are a virus-derived promoter and a ubiquitous expression promoter such as metallothionein promoter, but an NGF gene promoter, an enolase gene promoter, etc. that are expressed specifically in the brain are preferably employed.

The DNA of the invention is transfected into a fertilized egg in a manner such that the DNA is certainly present in all the germinal cells and somatic cells of the target animal. The fact that the protein of the invention is present in the germinal cells of the animal prepared by the DNA transfection means that all offsprings of the prepared animal will have the protein of the invention in all of the germinal cells and somatic cells thereof. The offspring of the animal that inherits the gene has the protein of the invention in all of the germinal cells and somatic cells thereof.

The animal, in which the DNA of the invention has been transfected, can be bred as the DNA-bearing animal under ordinary rearing environment, after confirming that the gene is stably retained through the mating. In addition, mating male with female animals, which have the target DNA, produces homozygote animals having the transfected gene in both homologous chromosomes, which can be bred by mating its male and female such that all the offsprings have the DNA.

The animal, in which the DNA of the invention is transfected, has the protein of the invention expressed in a high degree and hence, useful as an animal suitable for screening for an agonist or an antagonist of the protein of the invention.

The DNA-transfected animal of the invention can be used as a cell source of tissue culture. For example, the protein of the invention can be analyzed through a direct analysis of the DNA or RNA in the tissue of a mouse in which the DNA of the invention is transfected or through the analysis of a tissue in which the receptor protein of the invention is expressed by the gene. The cells from a tissue having the protein of the invention, cultured by the standard tissue culture technique, can be used for study of functions of cells derived from tissues such as a brain or a peripheral tissue, which are generally difficult to culture. Moreover, using the cells, it is possible to select a pharmaceutical to increase functions of various tissues. Further, it is also possible to isolate and purify the protein of the invention from a cell line with a high expression level, if any.

A test compound is added to the DNA-transfected animal of the invention for measurement of a heart performance, an electrocardiogram, a heart weight, etc. of the animal. The heart weight is a parameter of megalocardia. Specifically, the calculation of the heart weight per body weight, a left ventricle weight per body weight, and the left ventricle weight per right ventricle weight allows elucidation of the heart structure. Megalocardia occurred increases the aforementioned parameter, and therefore the test compound can be evaluated using a suppression of this increase as an index. After a test compound is administered to the DNA-transfected animal of the invention, cardiac infarction is induced by an surgery to measure the cardiac performance, electrocardiogram, heart weight, etc. of the animal. Moreover, by weighing an infarction layer after the cardiac infarction-inducing surgery, an activity of the test compound to suppress infarction development can be examined. A test compound is administered after the cardiac infarction-inducing surgery. Meanwhile, mating the animal with a genetic hypertension rat model, such as SHR rat, makes it possible to produce a new heart failure model. A test compound is administered to the heart failure model prepared in such way to examine the cardiac performance, electrocardiogram, heart weight, the infarction development-suppressing activity, etc. of the animal.

### [9] Knockout animals

The present invention provides a non-human mammal embryonic stem cell bearing the DNA of the invention inactivated and a non-human mammal deficient in expressing the DNA of the invention.

Thus, the present invention provides:
(1) a non-human embryonic stem cell in which the DNA of the invention is inactivated;
(2) the embryonic stem cell according to (1), in which the DNA is inactivated by introducing a reporter gene (e.g. β-galactosidase gene derived from Escherichia coli);
(3) the embryonic stem cell according to (1), which is resistant to neomycin;
(4) the embryonic stem cell according to (1), wherein the non-human mammal is a rodent;
(5) the embryonic stem cell according to (4), wherein the rodent is mouse;
(6) a non-human mammal deficient in expressing the DNA of the invention, in which the DNA of the invention is inactivated;
(7) the non-human mammal according to (6), in which the DNA is inactivated by introducing a reporter gene (e.g. β-galactosidase derived from Escherichia coli) therein and the reporter gene can be expressed under the control of a promoter for the DNA of the invention;
(8) the non-human mammal according to (6), which is a rodent;
(9) the non-human mammal according to (8), wherein the rodent is a mouse; and,
(10) a method of screening for a compound that enhances or inhibits the promoter activity for the DNA of the invention, which comprises administering a test compound to the mammal of (7) and detecting expression of the reporter gene.

The non-human mammal embryonic stem cell in which the DNA of the present invention is inactivated refers to the embryonic stem cells (abbreviated hereinafter as "ES cells") of a non-human mammal either in which the DNA expression ability is suppressed by the artificial mutation of the DNA of the invention present in the non-human mammal, or in which the activity of the polypeptide of the invention encoded by said DNA has substantially been eliminated so that the DNA is not substantially capable of expressing the polypeptide of the invention (sometimes referred to hereinafter as the knockout DNA of the present invention).

The non-human mammals used are similar to those as described above.

Techniques for artificially mutating the DNA of the invention include deletion of a part or all of the DNA sequence and insertion of or substitution with other DNA, by genetic engineering. The knockout DNA of the invention may be prepared by these mutations, for example, by shifting the reading frame of a codon or by disrupting the function of a promoter or exon.

Specifically, the non-human mammal embryonic stem cell in which the DNA of the invention is inactivated (abbreviated hereinafter as the ES cells with the DNA of the invention inactivated or the knocked-out ES cells of the invention) can be produced as follows. For example, the DNA of the invention that the target non-human mammal has is isolated, a drug-resistant gene, of which typical examples are neomycin-resistant, hygromycin-resistant or other drug-resistant genes, or a reporter gene or the like, of which typical examples are 1acZ (β-galactosidase gene) or cat (chloramphenicol acetyltransferase gene), is inserted into the exon to disrupt the function of the exon, or else a DNA sequence (such as polyA addition signal) which terminates the gene transcription is inserted into the intron between the exons to prevent synthesis of the complete mRNA. A DNA strand having the thus constructed DNA sequence to disrupt the gene (abbreviated hereinafter as "the targeting vector") is introduced into the chromosomes of the animal by homologous recombination. The knocked-out ES cell of the invention can be selected by analyzing the thus obtained ES cells either by the southern hybridization analysis using a DNA sequence on or near the DNA of the invention as a probe, or by the PCR analysis using as primers a DNA sequence on the targeting vector and a DNA sequence of a nearby region of the DNA of the invention used in producing the targeting vector.

The parent ES cells to inactivate the DNA of the invention by homologous recombination, etc. may be of a strain already established as described above, or may be originally established in accordance with a modification of the publicly known method by Evans and Kaufman. For example, in the case of mouse ES cells, currently it is common practice to use ES cells of the 129 strain, but their immunological genetic background is obscure. Accordingly, to establish another pure ES cell line, of which the immunological genetic background is clear, the C57BL/6 mouse or the BDF1 mouse (F1 hybrid between C57BL/6 and DBA/2), in which the low egg availability in the C57BL/6 mouse has been improved by crossing with DBA/2, may be preferably used. The BDF1 mouse is advantageous in that, when a pathologic model mouse is generated using the ES cells obtained therefrom, the genetic background can be changed to that of the C57BL/6 mouse by back-crossing with the C57BL/6 mouse, since its background is of the C57BL/6 mouse, as well as being advantageous in that egg availability is high and eggs are robust.

In establishing ES cells, blastocytes at 3.5 days after fertilization are commonly used. As well, embryos can be collected at the 8-cell stage, and cultured until the blastocyte stage to efficiently obtain a large number of early stage embryos.

Although the ES cells used may be of either sex, male ES cells are generally more convenient for generation of a germ chimera cell. It is also desirable that sex of the ES cells is determined as soon as possible to save painstaking culture time.

Methods for sex determination of the ES cell include the method in which a gene in the sex-determining region on the Y-chromosome is amplified by the PCR process and detected. When this method is used, one colony of ES cells (about 50 cells) is sufficient for sex-determination, while karyotype analysis requires about 10⁶ cells. Therefore, the first selection of ES cells at the early stage of culture can be based on sex determination, and male cells can be selected early, which saves a significant amount of time at the early stage of culture.

Second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical procedures, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1 to 10000 U/ml) on appropriate feeder cells such as STO fibroblasts, and treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By culturing ES cells to reach a high density in mono-layers or to form cell aggregates in suspension under appropriate conditions, they can differentiate to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the invention, which are obtained from the differentiated ES cells of the invention, are useful for an in vitro cell biological study of the function of the polypeptide of the invention.

The non-human mammal deficient in expression of the DNA of the invention can be distinguished from a normal animal by measuring the mRNA amount in the subject animal by a publicly known method, and indirectly comparing the levels of expression.

The non-human mammals used are similar to those as described above.

With respect to the non-human mammal deficient in expression of the DNA of the invention, the DNA of the invention can be knocked out by transfecting a targeting vector, prepared as described above, into mouse embryonic stem cells or egg cells thereof, and conducting homologous recombination in which the DNA sequence in the transfected targeting vector, wherein the DNA of the invention is inactivated, replaces the DNA of the invention on a chromosome of mouse embryonic stem cells or egg cells thereof.

The cells in which the DNA of the invention is knocked out can be identified either by the southern hybridization analysis using a DNA sequence on or near the DNA of the invention as a probe, or by the PCR analysis using as primers a DNA sequence on the targeting vector and another DNA sequence of a nearby region of the mouse-derived DNA of the invention used in creating the targeting vector. When using non-human mammal embryonic stem cells, a cell line in which the DNA of the invention is inactivated by homologous recombination can be cloned, and the cloned cells are injected into an embryo or blastocyst of a non-human mammal at an appropriate stage such as the 8-cell stage. The resulting chimera embryo is then transplanted to the uterus of the pseudopregnant non-human mammal. The resulting animal is a chimera animal comprising both cells having the normal locus of the DNA of the invention and the artificially mutated locus of the DNA of the invention.

When some germ cells of the chimera animal have a mutation on the locus of the DNA of the invention, an individual, whose entire tissue is composed of cells having a mutation on the locus of the DNA of the invention can be selected from a series of offsprings obtained by crossing such a chimera animal and a normal animal, e.g., by coat color identification, etc. The individuals thus obtained are normally deficient in heterozygous expression of the polypeptide of the invention. The individuals are deficient in homozygous expression of the polypeptide of the invention and can be obtained from offsprings of the intercross of the individuals deficient in heterozygous expression of the polypeptide of the invention.

When using egg cells, it is possible to obtain a transgenic non-human mammal having the targeting vector inserted into the chromosomes by microinjection of the DNA solution into an egg cell nucleus. From such transgenic non-human mammals, selected is one having the mutation on the DNA locus of the invention due to homologous recombination.

The animal in which the DNA of the invention has been knocked out in this way can be successively reared in a normal environment after confirmation that the DNA is knocked out in its offsprings obtained by breeding.

Reproductive lineages can also be obtained and maintained by ordinary methods. Thus, female and male animals having the inactivated DNA can be bred to obtain homozygote animals having the inactivated DNA in both loci of homologous chromosomes. The resulting homozygote animals can be efficiently reproduced by rearing under the condition of one normal individual and multiple homozygote individuals to a mother animal. By crossing male and female heterozygotes, homozygotes and heterozygotes having the inactivated DNA are successively reproduced.

The non-human mammal embryonic stem cell, in which the DNA of the invention is inactivated, is very useful for preparing a non-human mammal deficient in expression of the DNA of the invention.

Since the non-human mammal, in which the DNA of the invention is inactivated, lacks various biological activities derived from the polypeptide of the invention, such an animal can be a model for a disease resulted from inactivated biological activities of the polypeptide of the invention and thus, offers an effective study to investigate the causes for and therapy for these diseases.

### [9a] Method of screening a compound having a therapeutic/prophylactic effect on diseases caused by deficiency, damages, etc. of the DNA of the invention

The non-human mammal deficient in expression of the DNA of the invention can be employed for screening of a compound having a therapeutic/prophylactic effect on diseases caused by deficiency, damages, etc. of the DNA of the invention.

That is, the present invention provides a method of screening a compound having a therapeutic/prophylactic effect on diseases caused by deficiency, damages, etc. of the DNA of the invention, which comprises administering a test compound to the non-human mammal deficient in expression of the DNA of the invention and observing and measuring a change occurred in the animal.

As the non-human mammal deficient in expression of the DNA of the invention that can be employed for the screening method, the same examples as given hereinabove apply.

Examples of the test compound include peptides, proteins, non-peptide compounds, synthetic compounds, fermentation products, cell extracts, plant extracts, animal tissue extracts, blood plasma and the like and these compounds may be novel compounds or publicly known compounds.

Specifically, after treating the non-human mammal deficient in expression of the DNA of the present invention with a test compound, and making a comparison with an intact control animal, a change in each organ, tissue, disease conditions, etc. of the animal is used as an index to assess the therapeutic/prophylactic effects of the test compound.

The method of treating an test animal with a test compound includes oral administration, intravenous injection, etc., and it is appropriately selected depending upon conditions of the test animal, properties of the test compound, etc. In addition, the dose of the test compound can be appropriately selected depending on the administration route, nature of the test compound and the like.

For example, when the screening of the compound having preventive or therapeutic effect on diseases characterized by the cardiac hypofunction (e.g. heart diseases such as heart failure after cardiac infarction; angina pectoris; myocardosis; heart failure derived from diseases such as angina pectoris, myocardosis, etc.) is conducted, a test compound is added to the non-human mammal deficient in expression of the DNA of the invention to measure a heart performance, an electrocardiogram, a heart weight, etc. of the animal. The heart weight is a parameter of megalocardia. Specifically, the calculation of the heart weight per body weight, a left ventricle weight per body weight, and the left ventricle weight per right ventricle weight allows elucidation of the heart structure. Megalocardia occurred increases the aforementioned parameter, and therefore the test compound can be evaluated using a suppression of this increase as an index. After a test compound is administered to the non-human mammal deficient in expression of the DNA of the invention, cardiac infarction is induced by an surgery to measure the cardiac performance, electrocardiogram, heart weight, etc. of the animal. Moreover, by weighing an infarction layer after the cardiac infarction-inducing surgery, an activity of the test compound to suppress infarction development can be examined. A test compound is administered after the cardiac infarction-inducing surgery. Meanwhile, mating the animal with a genetic hypertension rat model, such as SHR rat, makes it possible to produce a new heart failure model. A test compound is administered to the heart failure model prepared in such way to examine the cardiac performance, electrocardiogram, heart weight, the infarction development-suppressing activity, etc. of the animal.

The compound obtained using the above screening method is a compound selected from the test compounds described above and exhibits a therapeutic and prophylactic effect on a disease caused by lack, damage, etc. of the polypeptide of the invention. Therefore, the compound can be employed as a safe and low toxic drug for the treatment and prevention of the disease. Furthermore, compounds derived from the compound obtained by the screening supra can be likewise employed.

The compound obtained by the screening method above may form a salt, and may be used in the form of a salt with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical composition comprising the compound obtained by the above screening method or a salt thereof may be manufactured in a manner similar to the method for preparing the composition comprising the protein of the invention as described above.

Since the pharmaceutical composition thus obtained is safe and low toxic, it can be administered to a human and other mammals (e.g. rat, mouse, guinea pig, rabbit, sheep, swine, bovine, horse, cat, dog, monkey).

A dose of the compound or its salt to be administered varies depending upon target disease, subject to be administered, route of administration, etc. For example, when the compound is orally administered, the compound is administered to an adult cancer patient (as 60 kg body weight) in a daily dose of about 0.1 to 100 mg, preferably about 1.0 to 50 mg, more preferably about 1.0 to 20 mg. For parenteral administration, a single dose may vary depending upon subject to be administered, target disease, etc. For example, when the compound is administered in the form of an injectable preparation, it is advantageous to administer the compound intravenously to an adult heart disease patient (as 60 kg body weight) in a daily dose of about 0.01 to 30 mg, preferably about 0.1 to 20 mg, more preferably about 0.1 to 10 mg. For other animals, the corresponding dose as converted per 60 kg body weight can be administered.

### [9b] Method for screening a compound that can enhance or inhibit the activity of the promoter for the DNA of the present invention

The present invention provides a method for screening a compound that can enhance or inhibit the activity of the promoter for the DNA of the invention or a salt thereof, which comprises administering a test compound to the non-human mammal deficient in expression of the DNA of the invention and detecting expression of the reporter gene.

In the screening method *supra*, used is the non-human mammal deficient in expression of the DNA of the invention in which the DNA of the invention is inactivated by introducing a reporter gene and the reporter gene is expressed under control of the promoter for the DNA of the invention.

Examples of the test compound are as described above.

Examples of the reporter gene are as described above. Preferably employed are β-galactosidase gene (lacZ), soluble alkaline phosphatase gene, luciferase gene and the like.

In the non-human mammal deficient in expression of the DNA of the invention wherein the DNA is substituted with the reporter gene, since the reporter gene is present under control of the promoter for the DNA of the invention, the activity of the promoter can be detected by monitoring the expression of the substance encoded by the reporter gene.

For example, when a part of the DNA region encoding the polypeptide of the invention is substituted with β-galactosidase gene (lacZ) derived from *Escherichia coli*, β-galactosidase is expressed in place of the polypeptide of the invention in a tissue where the polypeptide of the invention should originally be expressed. Thus, the expression state of the polypeptide of the invention can be readily observed in an animal body by staining with a reagent, e.g., 5-bromo-4-chloro-3-indolyl-β-galactopyranoside (X-gal) which is substrate for β-galactosidase. Specifically, a mouse deficient in the polypeptide of the invention, or its tissue section is fixed with glutaraldehyde, washed with phosphate buffered saline (PBS), and then incubated with a staining solution containing X-gal at room temperature or about 37°C for about 30 minutes to an hour. After the (3-galactosidase reaction is terminated by washing the tissue preparation with 1 mM EDTA/PBS solution, the color change is observed. Alternatively, the mRNA encoding 1acZ may be detected in a conventional manner.

The compound or a salt thereof obtained using the screening method *supra* is selected from the test compounds described above and can enhance or inhibit the promoter activity for the DNA of the invention.

The compound obtained by the screening method above may form a salt, and may be used in the form of a salt with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The compound or the salt thereof that can enhance the promoter activity for the DNA of the invention can enhance the expression of the polypeptide of the invention, and finally enhance the function of the polypeptide of the invention. Accordingly, it is useful as a therapeutic and/or prophylactic agent for diseases characterized by the cardiac hypofunction (e.g. heart diseases such as heart failure after cardiac infarction; angina pectoris; myocardosis; heart failure derived from diseases such as angina pectoris, myocardosis, etc.).

On the other hand, since the compound or the salt thereof that can inhibit the promoter activity for the DNA of the invention can inhibit the expression of the polypeptide of the invention, and finally inhibit the function of the polypeptide of the invention. Accordingly, it is useful as a therapeutic and/or prophylactic agent for diseases characterized by the cardiac hypofunction (e.g. heart diseases such as heart failure after cardiac infarction; angina pectoris; myocardosis; heart failure derived from diseases such as angina pectoris, myocardosis, etc.).

Furthermore, compounds derived from the compound obtained by the screening supra can be likewise employed.

A pharmaceutical composition comprising the compound or a salt thereof obtained by the screening method *supra* may be manufactured in a manner similar to the method for preparing the composition comprising the polypeptide of the invention as described above.

Since the pharmaceutical composition obtained is safe and low toxic, it can be administered to, for example, a human or other mammals (e.g., rat, mouse, guinea pig, rabbit, sheep, swine, bovine, horse, cat, dog, monkey, etc.)

A dose of the compound or its salt to be administered varies depending upon target disease, subject to be administered, route of administration, etc. For example, when the compound enhancing the promoter activity for the DNA of the invention is orally administered, the compound is administered to an adult heart disease patient (as 60 kg body weight) in a daily dose of about 0.1 to 100 mg, preferably about 1.0 to 50 mg, more preferably about 1.0 to 20 mg. For parenteral administration, a single dose varies depending upon subject to be administered, target disease, etc. For example, when the compound enhancing the promoter activity for the DNA of the invention is administered in the form of an injectable preparation, it is advantageous to administer the compound intravenously to an adult heart disease patient (as 60 kg body weight) in a daily dose of about 0.01 to 30 mg, preferably about 0.1 to 20 mg, more preferably about 0.1 to 10 mg. For other animals, the corresponding dose as converted per 60 kg body weight can be administered.

On the other hand, for example, when the compound inhibiting the promoter activity for the DNA of the invention is orally administered, the compound is administered to an adult heart disease patient (as 60 kg body weight) in a daily dose of about 0.1 to 100 mg, preferably about 1.0 to 50 mg, more preferably about 1.0 to 20 mg. For parenteral administration, a single dose varies depending upon subject to be administered, target disease, etc. For example, when the compound inhibiting the promoter activity for the DNA of the invention is administered in the form of an injectable preparation, it is advantageous to administer the compound intravenously to an adult heart disease patient (as 60 kg body weight) in a daily dose of about 0.01 to 30 mg, preferably about 0.1 to 20 mg, more preferably about 0.1 to 10 mg. For other animals, the corresponding dose as converted per 60 kg body weight can be administered.

As stated above, the non-human mammal deficient in expression of the DNA of the invention is extremely useful for screening the compound or its salt that enhances or inhibits the promoter activity to the DNA of the invention and can greatly contribute to elucidation of causes for various diseases related to deficiency in expression of the DNA of the invention and for the development of prophylactic/therapeutic drug for these diseases.

Furthermore, in case that a so-called transgenic animal (gene-transfected animal) is prepared by ligating various protein-coding genes downstream to a DNA sequence containing the promoter region for the protein of the present invention and injecting the same into an animal egg, it can be used to study the in vivo functions of such protein, which can be expressed in a specific manner. As well, in case that a cell line is established in which an appropriate reporter gene is ligated to the said promoter site, it can be used as a research system for a low-molecular weigh compound capable of enhancing or inhibiting specifically the in vivo production of the protein of the invention.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is selected unless otherwise indicated.
- DNA :: deoxyribonucleic acid
- cDNA :: complementary deoxyribonucleic acid
- A: adenine
- T: : thymine
- G: guanine
- C: : cytosine
- RNA :: ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP: : adenosine triphosphate
- EDTA :: ethylenediaminetetraacetic acid
- SDS: : sodium dodecyl sulfate
- Gly: glycine
- Ala: : alanine
- Val: : valine
- Leu: : leucine
- Ile: : isoleucine
- Ser: : serine
- Thr: threonine
- Cys: : cysteine
- Met: : methionine
- Glu: glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid

Substituents, protecting groups and reagents generally used in this specification are presented as the codes below.
- Me: : methyl
- Et: : ethyl
- Bu: : butyl
- Ph: : phenyl
- TC: thiazolidine-4(R)-carboxamido
- Tos: : p-toluenesulfonyl
- CHO :: formyl
- Bzl: : benzyl
- Cl₂-Bzl :: 2,6-dichlorobenzyl
- Bom :: benzyloxymethyl
- Z: benzyloxycarbonyl
- Cl-Z: : 2-chlorobenzyloxycarbonyl
- Br-Z: : 2-bromobenzyl oxycarbonyl
- Boc: : t-butoxycarbonyl
- DNP :: dinitrophenol
- Trt: : trityl
- Bum :: t-butoxymethyl
- Fmoc :: N-9-fluorenyl methoxycarbonyl
- HOBt :: 1-hydroxybenztriazole
- HOOBt :: 3,4-dihydro-3-hydroxy-4-oxo-1,2,3-benzotriazine
- HONB :: 1-hydroxy-5-norbornene-2,3-dicarboxyimide
- DCC :: N,N'-dichlorohexylcarbodiimide

The sequence identification numbers in the sequence listing of the specification indicates the following sequence, respectively.

### [SEQ ID NO: 1]

This shows the amino acid sequence of the protein encoded by a rat-derived novel gene according to the invention obtained in Example 1-3).

### [SEQ ID NO: 2]

This shows the base sequence of the rat-derived novel gene according to the invention obtained in Example 1.

### [SEQ ID NO: 3]

This shows the base sequence of the gene fragment obtained in Example 1.

### [SEQ ID NO: 4]

This shows the base sequence of the AP1 primer used in Example 1.

### [SEQ ID NO: 5]

This shows the base sequence of the primer used in Example 1.

### [SEQ ID NO: 6]

This shows the whole base sequence of the gene obtained in Example 1 and represented by SEQ ID No 2.

### [SEQ ID NO: 7]

This shows the base sequence of the primer used in Example 1.

### [SEQ ID NO: 8]

This shows the base sequence of the primer used in Example 1.

### [SEQ ID NO: 9]

This shows the base sequence of T7 primer used in Example 1.

### [SEQ ID NO: 10]

This shows the base sequence of SP6 primer used in Example 1.

### [SEQ ID NO: 11]

This shows the base sequence of the primer used in Example 2.

### [SEQ ID NO: 12]

This shows the base sequence of the primer used in Example 3.

### [SEQ ID NO: 13]

This shows the base sequence of the primer used in Example 3.

### [SEQ ID NO: 14]

This shows the base sequence of a fluorescent probe used in Example 3.

The transformant Escherichia coli DH5α/pTB2169, obtained in Example 1 described later, has been deposited in the Institute for Fermentation, Osaka (IFO), located at 2-17-85, Jyuso-Honmati, Yodogawa-ku, Osaka-shi, Osaka, 532-8686, Japan, under Accession Number IFO 16480 since September 26, 2000; and in the International Patent Organism Depositary, the National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki, 305-8566, Japan, under Accession Number FERM BP-7331 since October 19, 2000.

### EXAMPLES

The present invention is described in detail below with reference to Examples, but not intended to limit the scope of the present invention thereto. The gene manipulation procedures using Escherichia coli were performed according to the methods described in "Molecular Cloning", 2nd ed., J. Sambrook et al., Cold Spring Harbor Lab. Press, 1989.

### Example 1:

### (1) Preparation of a rat model of cardiac infarction

Male Wistar rats (11-week-old; body weight 300 to 400 g) were anesthetized with pentobarbital (50 mg/kg, i.p.) according to the report by Watanabe et al. (Circulation Res. 69: 370-377, 1991) and subjected to the median sternotomy under artificial respiration. After incision of a pleuropericardial membrane, a heart was exposed. The coronary artery together with cardiac muscles was ligated in the origin of a left anterior descending branch of the coronary artery using a surgical needle with silk suture (Elp, 5-0 silk) and then the incision of the chest was closed. For a sham surgery group, the chest was closed without the suture ligature of the coronary artery. After recovery from anesthesia, all rats were subjected to a normal keeping.

### (2) Extraction of total RNA

At 1 week, 8 weeks, 20 weeks, and 30 weeks passed after the surgery, these rats were subjected to thoracotomy under pentobarbital anesthesia, and hearts were isolated. Blood was washed out from the coronary artery by retrograde perfusion with saline from an aorta. Tissues other than the left ventricle were removed from the excised heart using scissors and then formation of infarction was observed followed by removal of the infarction region (region where a scar has been formed) to leave a non-infarction region. This region was minced with scissors and the total RNA was extracted using Isogen (Wako Pure Chemicals) therefrom..

### (3) Cloning of novel rat RGS5-analogous gene

A DNA degradation was carried out to remove a genome DNA from the total RNA by using "Enzyme Set for DD" (Takara) and then differential display (DD) analysis was conducted using "Fluorescence Differential Display Kit: Fluorescein Version" (Takara). The total RNA derived from the left ventricle 8 weeks after the sham surgery as a control tissue was used.

As a result, in comparison with the control tissue, a band was found, which was remarkably increased in the tissue at 1 week, 20 weeks, and 30 weeks after the surgery, and decreased in the tissue at 8 weeks after the surgery. This band was cut out from the acrylamide gel with a cutter knife, suspended in sterilized distilled water, and heated at 95 °C for 10 minutes to extract the gene fragment from the gel. Then, the gene fragment was amplified again by PCR to determine its DNA sequence. On the basis of the base sequence (SEQ ID No: 3) obtained in this way, homology search was carried out by BlastN program on a public database or the Geneble Database, indicating that the base sequence has homology as high as about 96% with the mouse G-protein signal regulator (mouse RGS5; GenBank accession number: U67188).

Subsequently, on the basis of SEQ ID No: 3, full-length cloning of the gene fragment was carried out by 5'-RACE method.

A PCR was carried out using "Marathon Ready Heart cDNA Library" (Clontech) as a template, and using two primer DNAs, i.e. AP1 primer (SEQ ID No: 4) and a primer having the base sequence represented by SEQ ID No: 5 to clone the 5'-upstream region of the gene fragment. Determination of the base sequence of the PCR product showed that it has the base sequence (SEQ ID No: 6) containing an open reading frame (ORF). On the basis of this sequence, a PCR was carried out using 2 primer DNAs, i.e. a 5'-upstream primer (SEQ ID No: 7) and a 3'-downstream primer (SEQ ID No: 8) to obtain the ORF (SEQ ID No: 2). The PCR reaction was conducted using Pfu DNA polymerase (Toyobo) on Thermal Cycler Gene Amp PCR System 9700 (Perkin Elmer) with 33 cycles each consisting of reactions at 95 °C for 10 seconds, at 60 °C for 30 seconds, and at 72 °C for 3 minutes.

The thus obtained DNA having the base sequence represented by SEQ ID No: 2 was subcloned into pCR II-Blunt TOPO vector by using Zero Blunt TOPO PCR Cloning Kit (Invitrogen) and the resulting plasmid was named pTB2169. In addition, the base sequence thereof was determined on Fluorescence DNA Sequencer (ABI Prism 377, Perkin Elmer) by the reaction using "Cycle Sequence Kit" (PE Applied BioSystyems) and the publicly known synthetic primers [T7 primer (SEQ ID No: 9) and SP6 primer (SEQ ID No: 10)], confirming that the DNA is a gene encoding a new RGS protein. The aforementioned pTB2169 was transfected into Escherichia coli to prepare a transformant, named E. coli DH5α/pTB2169.

It was further found that the protein (181 amino acid residues represented by SEQ ID No: 1) encoded by the DNA having the base sequence represented by SEQ ID No: 2 differs from the publicly known mouse RGS5 (181 amino acid residues) in 7 residues in the amino acid sequences.

### Example 2:

### Analysis of tissue distribution of the gene in a normal rat

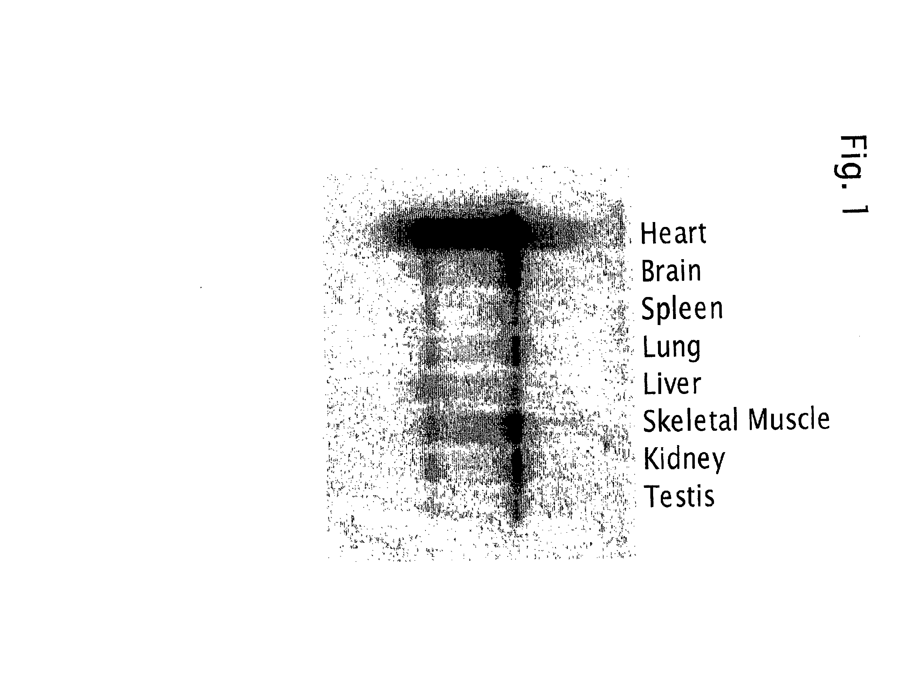

In order to obtain a probe for the northern blotting analysis, a PCR was carried out using cDNA having the base sequence represented by SEQ ID No: 2, obtained in Example 1, as the template, and using a primer having the base sequence represented by SEQ ID No: 8 and another primer having the base sequence represented by SEQ ID No: 11 in the same way as described in Example 1-(3). The "Rat MTN Blot" membrane (Clontech) was used for the northern blotting analysis. Prehybridization was conducted at 68 °C in "Express Hyb Hybridization Solution" (Clontech) as a hybridization solution. Meanwhile, the rat RGS-like gene fragment prepared above as the probe was labeled with [α-³²P] dCTP using "BcaBEST Labeling Kit" (Takara). Hybridization was conducted in "Express Hyb Hybridization Solution" (Clontech) containing the labeled probe at 68 °C for 1 hour. The membrane was finally washed with a solution of 0.1x SSC and 0.1% SDS at 50 °C, and BAS-2000 (Fuji Film) was used for detection. The result is shown in Fig. 1.

The result shows that the novel RGS gene (SEQ ID No: 2) of the invention is mainly expressed in heart. Moreover, a certain level of the expression was observed in brain and skeletal muscle.

### Example 3:

### Analysis of changes of the gene expression with time in a rat model of cardiac infarction

cDNAs were synthesized using TaqMan Reverse Transcription Reagents (PE Applied BioSystyems) from the total RNAs derived from the non-infarction regions of the rat left ventricles at 1 week, 8 weeks, 20 weeks, and 30 weeks after the surgery inducing cardiac infarction, which is described in Example 1-(2), and from the total RNA derived from the left ventricles at 8 weeks after the sham surgery, used as the control.

Subsequently, the copy number of the cDNA having the base sequence represented by SEQ ID No: 2 was determined on ABI Prism 7700 Sequence Detection System, by PCR using the DNAs having the base sequences represented by SEQ ID No: 12 and SEQ ID No: 13 as primers and using a fluorescent-labeled form of the DNA having the base sequence represented by SEQ ID No: 14 (PE Applied BioSystyems) as the probe. This reaction was conducted by using "TaqMan PCR Core Reagents Kit" (PE Applied BioSystyems) and following an instruction manual attached. A method for preparation of a standard used for the quantification is described below.

The cDNA was synthesized from the total RNA derived from the non-infarction region of the rat left ventricle at 1 week after the surgery inducing cardiac infarction, by using TaqMan Reverse Transcription Reagents (PE Applied BioSystyems). A PCR was then conducted using the DNAs having the base sequences represented by SEQ ID No: 12 and SEQ ID No: 13 as primers, and the thus obtained gene fragment having a partial sequence of the base sequence represented by SEQ ID No: 2 was referred to the standard. Further, the determined copy number was corrected using as an internal control, the copy number of glycerol-3-phosphate dehydrogenase determined in the same way as the determination of the copy number of the cDNA having the base sequence represented by SEQ ID No: 2, and then was expressed as the change ratio over the copy number of the control tissue.

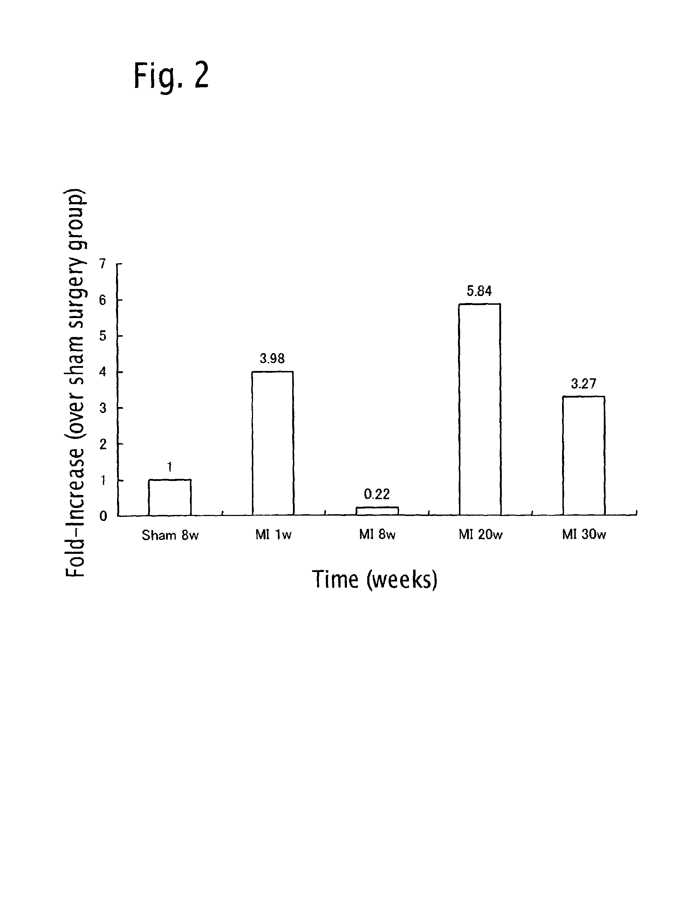

The results were shown in Fig. 2. The correction was made by dividing the copy number (the expression amount) of the cDNA having the base sequence represented by SEQ ID No: 2 in the left ventricle by the copy number of the housekeeping gene or glycerol-3-phosphate dehydrogenase gene. The ordinate axis represents in "folds increase" the change ratio of the corrected copy number over the measurement in the sham surgery group. The abscissa axis represents names of samples collected with time from the heart failure rat model used. "Sham 8w" shows the sample analyzed for the heart of the sham surgery group; "MI 1w" shows the sample analyzed for the heart at 1 week after the surgery; "MI 8w" shows the sample analyzed for the heart at 8 weeks after the surgery; "MI 20w" shows the sample analyzed for the heart at 20 weeks after the surgery; and "MI 30w" shows the sample analyzed for the heart at 30 weeks after the surgery.

This figure makes clear that the expression of the cDNA having the base sequence represented by SEQ ID No: 2 was increased (3.98 folds) at 1 week after the surgery, then remarkably decreased (0.22 folds) at 8 weeks after the surgery, and increased again (5.84 folds and 3.27 folds) at 20 weeks and 30 weeks after the surgery, respectively.

The time of 1 week just after the surgery is considered as the time when infarction is being formed and it is presumed that myocardial cells rapidly die and are stripped off at a downstream region of the ligated coronary artery, where lymphocytes are infiltrating and inflammation is developed. In view of that the time from 20 to 30 weeks after the surgery is just before the time when death cases are found, it is presumed that the time of 8 weeks after the surgery is the time when the compensation mechanisms are working and that the time following 20 week after the surgery is the time when the compensation mechanisms are insufficiently working or excessively working to result in the compensation failure. Therefore, the time following 1 week after the surgery is regarded as an acute stage, the time following 8 weeks after the surgery as a chronic stage, and the time following 20 weeks after the surgery as an end stage.

The compensation mechanisms related to transition from cardiac infarction to heart failure are presumed as follows. Loss (necrosis or apoptosis) of some of the myocardial cells induces hypertrophy of the remaining myocardial cells such that the whole heart can compensate for decreased functions due to the loss of myocardial cells, finally resulting in reconstruction of the heart (cardiac remodeling) accompanying cardiac dilatation and fibrosis. In this way, the cardiac performance is functionally compensated. On the other hand, it is indicated that the cardiac remodeling or the excessive compensation by itself has a risk of inducing the heart failure (Naika (Internal Medicine) 79: 2-20, 1997). However, no molecule relating to the compensation failure per se has been identified and thus its mechanism is not still evident.

The expression of the cDNA having the base sequence represented by SEQ ID No: 2, obtained in Example 1, is remarkably decreased at the chronic stage and increased at the end stage. Therefore, it is expected that the gene may be involved in the cardiac compensation mechanisms and the compensation failure and thus that such pathological conditions may be improved by adjusting the expression of the gene. Suitable controlling of expression of this gene allows suppression of the excessive compensation and the compensation failure (the decreased expression of the gene induces in excess the compensation mechanisms and the increased expression of the gene accelerates the compensation failure) and thus this is useful as a preventive and a remedy for a heart disease.

### INDUSTRIAL APPLICABILITY

The protein or its salt comprising the same or substantially the same amino acid sequence as the amino acid sequence represented by SEQ ID NO: 1 is novel. The compound regulating the activity of the protein or its salt and the antibody regulating the activity of the protein or its salt can be used, for example, as a preventive and therapeutic drug for heart diseases. The antisensenucleotide having a base sequence complementary or substantially complementary to the DNA encoding the protein or its salt can suppress the expression of the protein or its salt and can be used, for example, as a preventive and therapeutic drug for heart diseases. The preparation of the DNA-transfected animal and the knockout animal of the invention is useful for a cause-effect study on heart diseases and its associated diseases and for a preventive and therapeutic method for these diseases.

## Claims

1. A protein having the same or substantially the same amino acid sequence as the amino acid sequence shown by SEQ ID NO: 1, or a salt thereof.

2. A protein comprising the amino acid sequence shown by SEQ ID NO: 1, or a salt thereof.

3. A partial peptide of the protein according to claim 1, or a salt thereof.

4. A polynucleotide comprising a polynucleotide encoding the protein according to claim 1 or its salt, or the partial peptide according to claim 3 or its salt.

5. The polynucleotide according to claim 4 which is a DNA.

6. The DNA according to claim 5 which comprises the base sequence represented by SEQ ID NO: 2.

7. A recombinant vector comprising the polynucleotide according to claim 4.

8. A transformant transformed by the recombinant vector according to claim 7.

9. A method of manufacturing the protein according to claim 1 or its salt or the partial peptide according to claim 3 or its salt, which comprises culturing the transformant according to claim 8 to produce and accumulate the protein according to claim 1 or its salt or the partial peptide according to claim 3 or its salt; and collecting it.

10. A pharmaceutical composition comprising the protein according to claim 1 or its salt, or the partial peptide according to claim 3 or its salt.

11. An antibody to the protein according to claim 1 or its salt, or the partial peptide according to claim 3 or its salt.

12. A diagnostic agent comprising the antibody according to claim 11.

13. A method of screening for a compound or its salt regulating an activity of the protein according to claim 1 or its salt, or the partial peptide according to claim 3 or its salt, which comprises using the protein according to claim 1 or its salt, or the partial peptide according to claim 3 or its salt.

14. The screening method according to claim 13 wherein the protein according to claim 1 or its salt, or the partial peptide according to claim 3 or its salt is expressed in a cytoplasm of a transformant transformed by a DNA comprising a DNA encoding the protein according to claim 1 or its salt or the partial peptide according to claim 3 or its salt.

15. A kit for screening for a compound or its salt regulating the activity of the protein according to claim 1 or its salt, or the partial peptide according to claim 3 or its salt, which comprises the protein according to claim 1 or its salt, or the partial peptide according to claim 3 or its salt.

16. A compound or its salt regulating the activity of the protein according to claim 1 or its salt, or the partial peptide according to claim 3 or its salt, which is obtained by the screening method according to claim 13 or the screening kit according to claim 15.

17. A pharmaceutical composition comprising the compound according to claim 16 or its salt.

18. The pharmaceutical composition according to claim 17, which is a preventive and/or therapeutic agent for a heart disease.

19. An antisense nucleotide having a base sequence complementary or substantially complementary to a DNA encoding the protein according to claim 1 or its salt or the partial peptide according to claim 3 or its salt.

20. A pharmaceutical composition comprising the antisense nucleotide according to claim 19.

21. A pharmaceutical composition comprising the polynucleotide according to claim 4.

22. A diagnostic agent comprising the polynucleotide according to claim 4.

23. A preventive and/or therapeutic method for a heart disease in a mammal, which comprises administering an effective amount of the compound according to claim 16 or its salt to the mammal.

24. A use of the compound according to claim 16 or its salt for manufacturing a preventive and/or therapeutic agent for a heart disease.


SEQUENCE LISTING

<110> Takeda Chemical Industries, Ltd.

<120> Novel disease-associated gene and use thereof

<130> P01-0237PCT

<140> PCT/JP01/09140
<141> 2001-10-18

<150> JP 2000-319912
<151> 2000-10-19

<150> JP 2000-350183
<151> 2000-11-16

<160> 14

<210> 1
<211> 181
<212> PRT
<213> Rat

<400> 1
Met Cys Lys Gly Leu Ala Ala Leu Pro His Ser Cys Leu Glu Arg Ala
                5                   10                  15
Lys Glu Ile Lys Ile Lys Leu Gly Ile Leu Leu Gln Lys Pro Asp Ser
            20                  25                  30
Ala Val Asp Leu Val Ile Pro Tyr Asn Glu Lys Pro Glu Lys Pro Ala
        35                  40                  45
Lys Ala His Lys Pro Ser Leu Glu Glu Val Leu Gln Trp Arg Gln Ser
    50                  55                  60
Leu Asp Lys Leu Leu Gln Ser Asn Tyr Gly Phe Ala Ser Phe Lys Ser
65                  70                  75                  80
Phe Leu Lys Ser Glu Phe Ser Glu Glu Asn Leu Glu Phe Trp Val Ala
                85                  90                  95

Cys Glu Asn Tyr Lys Lys Ile Lys Ser Pro Ile Lys Met Ala Glu Lys
            100                 105                 110
Ala Lys Gln Ile Tyr Glu Glu Phe Ile Gln Thr Glu Ala Pro Lys Glu
        115                 120                 125
Val Asn Ile Asp His Phe Thr Lys Asp Ile Thr Met Lys Asn Leu Val
    130                 135                 140
Glu Pro Ser Pro His Ser Phe Asp Leu Ala Gln Lys Arg Ile Tyr Ala
145                 150                 155                 160
Leu Met Glu Lys Asp Ser Leu Pro Arg Phe Val Arg Ser Glu Phe Tyr
                165                 170                 175
Lys Glu Leu Ile Asn
            180

<210> 2
<211> 543
<212> DNA
<213> Rat

<400> 2
atgtgtaagg gactggcagc tctgccacac tcatgcctgg aaagggccaa agagatcaag 60
atcaaattgg gtattcttct ccagaagcca gactctgctg ttgaccttgt cattccatat 120
aatgagaagc cggagaagcc tgccaaggcg cacaagccct cgctggagga ggtcctgcaa 180
tggcgccaat ccctggacaa acttctccag agcaactacg gatttgccag cttcaaaagt 240
ttcctgaagt ctgaattcag tgaggaaaac cttgagttct gggttgcctg tgagaattac 300
aagaagatca agtcccccat caaaatggca gagaaggcaa agcaaatcta tgaagaattc 360
atccagacag aggcccctaa agaggtgaac attgaccact tcactaaaga catcaccatg 420
aagaacctgg tggaaccttc ccctcacagc tttgacctgg cccagaaaag gatctacgcc 480
ctgatggaga aggattctct gccccgcttc gtgcgctctg agttttataa ggagttaatc 540
aac 543

<210> 3
<211> 378
<212> DNA
<213> Rat

<400> 3
atcaaaatgg cagagaaggc aaagcaaatc tatgaagaat tcatccagac agaggcccct 60

aaagaggtga acattgacca cttcactaaa gacatcacca tgaagaacct ggtggaacct 120
tcccctcaca gctttgacct ggcccagaaa aggatctacg ccctgatgga gaaggattct 180
ctgccccgct tcgtgcgctc tgagttttat aaggagttaa tcaactagta attgggtcag 240
gcatcaaaaa gtcaccctgt gagttgagtt acatcctcta gagcagtaca gcatccccta 300
ggcacctgca catttctcca tagcagcttt gctccaagac acccaaacat aggcgaacca 360
caggccgtgt tgctaact 378

<210> 4
<211> 27
<212> DNA
<213> Artificial Sequence

<220>
<223> AP1 Primer

<400> 4
ccatcctaat acgactcact atagggc 27

<210> 5
<211> 20
<212> DNA
<213> Artificial Sequence

<220>
<223> Primer

<400> 5
agttagcaac acggcctgtg 20

<210> 6
<211> 817
<212> DNA
<213> Rat

<400> 6
tccatcataa tacgactcac tatagggctc gagcgccgcc cgggcaggtg tttccacaga 60
cttacttgtt cgtctgttga gaggttcgtg ctcaagttga ggacttgagc cgcgcgccaa 120

aatgtgtaag ggactggcag ctctgccaca ctcatgcctg gaaagggcca aagagatcaa 180
gatcaaattg ggtattcttc tccagaagcc agactctgct gttgaccttg tcattccata 240
taatgagaag ccggagaagc ctgccaaggc gcacaagccc tcgctggagg aggtcctgca 300
atggcgccaa tccctggaca aacttctcca gagcaactac ggatttgcca gcttcaaaag 360
tttcctgaag tctgaattca gtgaggaaaa ccttgagttc tgggttgcct gtgagaatta 420
caagaagatc aagtccccca tcaaaatggc agagaaggca aagcaaatct atgaagaatt 480
catccagaca gaggccccta aagaggtgaa cattgaccac ttcactaaag acatcaccat 540
gaagaacctg gtggaacctt cccctcacag ctttgacctg gcccagaaaa ggatctacgc 600
cctgatggag aaggattctc tgccccgctt cgtgcgctct gagttttata aggagttaat 660
caactagtaa ttgggtcagg catcaaaaag tcaccctgtg agttgagtta catcctctag 720
agcagtacag catcccctag gcacctgcac atttctccat agcagctttg ctccaagaca 780
cccaaacata ggcgaaccac aggccgtgtt gctaact 817

<210> 7
<211> 21
<212> DNA
<213> Artificial Sequence

<220>
<223> Primer

<400> 7
cgggcaggtg tttccacaga c 21

<210> 8
<211> 22
<212> DNA
<213> Artificial Sequence

<220>
<223> Primer

<400> 8
ggtgtcttgg agcaaagctg ct 22

<210> 9
<211> 17

<212> DNA
<213> Artificial Sequence

<220>
<223> T7 Primer

<400> 9
taatacgact cactata                                17

<210> 10
<211> 18
<212> DNA
<213> Artificial Sequence

<220>
<223> SP6 Primer

<400> 10
atttaggtga cactatag                               18

<210> 11
<211> 24
<212> DNA
<213> Artificial Sequence

<220>
<223> Primer

<400> 11
atcaaaatgg cagagaaggc aaag                        24

<210> 12
<211> 21
<212> DNA
<213> Artificial Sequence

<220>

<223> Primer

<400> 12
tgacctggcc cagaaaagga t                21

<210> 13
<211> 21
<212> DNA
<213> Artificial Sequence

<220>
<223> Primer

<400> 13
aaaactcaga gcgcacgaag c                21

<210> 14
<211> 28
<212> DNA
<213> Artificial Sequence

<220>
<223> Probe

<400> 14
tacgccctga tggagaagga ttctctgc         28